# EUROPÄISCHE PATENTSCHRIFT

(11) **EP 2 640 340 B1**
(45) Veröffentlichungstag und Bekanntmachung des Hinweises auf die Patenterteilung: **31.12.2014**
(21) Anmeldenummer: 11784475.3
(22) Anmeldetag: 11.11.2011
(51) Int. Cl.: A61J 7/00

(54) **DOSIERVORRICHTUNG**
DOSING DEVICE
DISPOSITIF DE DOSAGE

(30) Priorität: 18.11.2010 DE 102010044141
(43) Veröffentlichungstag der Anmeldung: 25.09.2013
(73) Patentinhaber: Raumedic AG, 95213 Münchberg (DE)
(72) Erfinder: JAKOB, Thomas, 95100 Selb (DE); SKAPER, Frank, 95191 Leupoldsgrün (DE); KELLNER, Thorsten, 95445 Bayreuth (DE); RICHTER, Frank, 07366 Harra (DE)
(74) Vertreter: Rau, Schneck & Hübner Patentanwälte Rechtsanwälte PartGmbB
(86) Internationale Anmeldenummer: PCT/EP2011/069956
(87) Internationale Veröffentlichungsnummer: WO 2012/065921

(56) Entgegenhaltungen:
- WO-A1-2010/043317
- WO-A1-2010/122981
- WO-A2-2008/122438
- US-A1- 2007 017 890

## Beschreibung

Die Erfindung betrifft eine Dosiervorrichtung zur dosierten Verabreichung eines fließfähigen Präparates zusammen mit einem fließfähigen Trägermedium.

Eine derartige Dosiervorrichtung ist bekannt aus der WO 2010/043 317 A1. Eine weitere Dosiervorrichtung ist bekannt aus der WO 2008/122 438 A2. Aus der DE 30 16 772 C2 ist eine Spritze zur Probenentnahme bekannt.

Es ist eine Aufgabe der vorliegenden Erfindung, eine Dosiervorrichtung der eingangs genannten Art benutzerfreundlich und möglichst kompakt zu gestalten.

Diese Aufgabe ist erfindungsgemäß gelöst durch eine Dosiervorrichtung mit den im Anspruch 1 angegebenen Merkmalen.

Erfindungsgemäß wurde erkannt, dass über das Konzept eines mehrteiligen Dosier-Kolbenkörpers ein neuer Freiheitsgrad entsteht, der insbesondere dazu benutzt werden kann, einen Präparatfluss vom Inneren des Vorratsbehälters in das Innere des Dosier-Kolbenkörpers wirksam zu unterbinden, wenn dies nicht gewünscht ist. Der Präparatfluss vom Inneren des Vorratsbehälters in das Innere des Dosier-Kolbenkörpers kann unabhängig von einer Druckdifferenz zwischen diesen beiden angrenzenden Räumen wahlweise unterbunden und ermöglicht werden. Die Dosiervorrichtung kann prinzipiell aus nur drei Einzelteilen ausgeführt sein, nämlich dem Vorratsbehälter und einem zweiteiligen Dosier-Kolbenkörper mit einer Dosierhülse und einem inneren Kolbenkörper. Die Herstellung einer Durchgangsöffnung durch Verdrehen des inneren Kolbenkörpers zu dieser umgebenden Dosierhülse schafft die Möglichkeit, in der Durchgangs-Relativ-Drehposition einen Durchgang mit relativ großer Durchgangsweite zu erzeugen, was die Benutzung der Dosiervorrichtung nach abgeschlossener Dosierung, beim Ansaugen des Präparates durch die Ansaugöffnung erleichtert. Das Totvolumen innerhalb der Dosiervorrichtung kann im Vergleich zu bekannten Gestaltungen reduziert sein. Solange die Dosiervorrichtung in der Verschluss-Relativ-Drehposition der Dosierhülse zum inneren Kolbenkörper vorliegt, kann die Dosiervorrichtung wie eine Spritze genutzt werden. Mit dem Kolbenkörper kann dann ein fließfähiges Präparat in den Vorratsbehälter wie beim Aufziehen einer Spritze eingesogen und über die Zugangsöffnung im Vorratsbehälter wieder abgegeben, beispielsweise oral appliziert werden.

Eine Rasteinrichtung nach Anspruch 2 ermöglicht eine bequeme Präparatdosierung.

Eine Ausgestaltung der Dosierhülse nach Anspruch 3 ermöglicht eine kompakte Gestaltung auch dann, wenn die Rasteinrichtung, z. B. zur Vermeidung von Undichtigkeiten, getrennt von Dichtflächen zwischen dem Dosier-Kolben-Körper und dem Vorratsbehälter angeordnet ist. Die Dosiervorrichtung mit dem inneren und dem äußeren Dosierhülsenabschnitt kann insbesondere längs der Längsachse der Dosiervorrichtung mit vorteilhaft geringer Baulänge gestaltet sein. Der innere und der äußere Dosierhülsenabschnitt können einander längs der Längsachse zumindest abschnittsweise oder auch vollständig überlappend angeordnet sein. Die Rasteinrichtung im Bereich des äußeren Dosierhülsenabschnitts kann von außen gut zugänglich und insbesondere von außen sichtbar gestaltet werden, was die Ablesbarkeit beim Dosieren vereinfacht. Es kann beispielsweise eine von außen ablesbare Skalierung im Bereich der Rasteinrichtung vorgesehen sein.

Eine einstückig angeformte Dichtung nach Anspruch 4 vereinfacht die Montage der Dosiervorrichtung und kann zu einer Verringerung der Herstellkosten insgesamt führen.

Ein Dichtkörper nach Anspruch 5 verbessert die dichtende Wirkung zwischen dem Dosierhülsenboden und dem Kolbenkörperboden dann, wenn der innere Kolbenkörper relativ zur Dosierhülse in die Verschluss-Relativ-Drehposition gedreht ist. Der Dichtkörper kann als separates Bauteil gestaltet sein.

Ein Einwegeventil nach Anspruch 6 verhindert, dass bereits angesaugtes Präparat unerwünscht wieder aus dem Inneren des inneren Kolbenkörpers zurück in das Innere des Vorratsbehälters gelangt.

Die Funktion des vorstehend angesprochenen Dichtkörpers und die Funktion des vorstehend angesprochenen Einwegeventils können von ein und derselben Komponente erfüllt werden.

Eine Anordnung des Dichtkörpers bzw. des Einwegeventils nach Anspruch 7 ermöglicht eine kompakte Gestaltung.

Eine Drehführung nach Anspruch 8 ermöglicht eine saubere Definition einerseits der Durchgangs-Relativ-Drehposition und andererseits der Verschluss-Relativ-Drehposition sowie eine saubere Definition einer Verlagerungs-Drehbewegung der äußeren Dosierhülse zum inneren Kolbenkörper zwischen diesen beiden Drehpositionen.

Eine Anschlageinrichtung nach Anspruch 9 ermöglicht eine Präparatdosierung, ohne dass es erforderlich ist, eine Skala abzulesen.

Eine Ausgestaltung der Anschlageinrichtung nach Anspruch 10 ermöglicht eine Anordnung des Anschlagkörpers, die von außen zugänglich ist. Auf diese Weise kann der Anschlagkörper manuell zur Vorgabe einer Dosiermenge verstellbar gestaltet sein.

Eine Verschiebbarkeit des Anschlagkörpers nach Anspruch 11 ist eine unaufwendige Variante zur Vorgabe jeweils einer benötigten Dosierstellung. Das Langloch kann im äußeren Dosierhülsenabschnitt der Dosierhülse ausgeführt sein. Ein Ende des Langlochs kann gleichzeitig den Gegenanschlagkörper darstellen.

Eine Verriegelungsgestaltung nach Anspruch 12 führt zu einer Axialsicherung des Anschlagkörpers. Die Verriegelungsstruktur und die Verriegelungs-Gegenstruktur können durch Verzahnungen oder durch Gewindestrukturen oder andere zueinander komplementäre Strukturen gebildet sein.

Rasteinrichtungen nach Anspruch 13 ermöglichen eine definierte Umstellung des Anschlagkörpers von der Freigabestellung in die Verriegelungsstellung.

Eine zweiteilige Gestaltung des Vorratsbehälters nach Anspruch 14 erlaubt, beispielsweise bei Herstellung dieser Komponenten im Spritzgussverfahren, eine größere Gestaltungsfreiheit bei der Formgebung. Die Drehsicherung gegen eine unerwünschte Relativdrehung des Dosierzylinders zur Führungshülse um die Längsachse kann durch eine Radialnut gegeben sein, die im Dosierzylinder oder in der Führungshülse ausgeführt ist und die in eine komplementär ausgeführte Radialrippe, die wiederum an der Führungshülse oder am Dosierzylinder ausgeführt ist, eingreift. Die Axialsicherung des Dosierzylinders an der Führungshülse kann durch eine umlaufende Ring- oder Umfangsrippe gebildet sein, die am Dosierzylinder oder an der Führungshülse ausgebildet ist und in eine komplementär ausgeführte Umfangsnut eingreift, die wiederum in der Führungshülse oder im Dosierzylinder ausgeführt ist. Alternativ kann die Drehsicherung des Dosierzylinders an der Führungshülse durch nicht rotationssymmetrisch gestaltete und zueinander komplementäre Umfangs- oder Stirnwände des Dosierzylinders und/oder der Führungshülse gebildet sein, die insbesondere als Drehsicherungs-Wandabschnitte ausgeführt sind. Die Axial- und Drehsicherung kann, eine entsprechende Gestaltung der Verriegelungsstrukturen und Verriegelungs-Gegenstrukturen für den Anschlagkörper vorausgesetzt, zur Vorgabe einer Dosierstellung des Anschlagkörpers genutzt werden.

Eine Axial/Drehsicherungseinrichtung nach Anspruch 15 ist in der Handhabung einfach und in der Herstellung wenig aufwendig. Diese Sicherungseinrichtung kann wiederum eine Rasteinrichtung nach Art einer Umfangsrippe aufweisen, die komplementär zu einer Umfangsnut gestaltet ist.

Gemäß einem weiteren Aspekt ist die eingangs genannte Aufgabe gelöst durch eine Dosiervorrichtung zur dosierten Verabreichung eines fließfähigen Präparates zusammen mit einem fließfähigen Trägermediums,
- mit einem Vorratsbehälter für das Präparat, der über mindestens eine Zugangsöffnung mit der Umgebung kommuniziert,
- mit einem Dosier-Kolbenkörper, der zumindest abschnittsweise in den Vorratsbehälter (2) abgedichtet verlagerbar ist zwischen
   -- einer Ausgangsposition, in der der Dosier-Kolbenkörper ganz in den Vorratsbehälter eingeschoben ist und
   -- mindestens einer Dosierposition, in der der Dosier-Kolbenkörper bis zu einer der gewünschten Dosiermenge des Präparats entsprechenden Wegstrecke aus dem Vorratsbehälter herausgeschoben ist.

Der Dosier-Kolbenkörper ist dabei insbesondere nicht hohl, sondern als Vollkörper ausgeführt. Die Dosiervorrichtung ist also nach Art einer Dosierspritze gestaltet, wobei über den Kolbenkörper das zu dosierende fließfähige Präparat in den Vorratsbehälter eingesaugt und Dosierpositionen des Dosier-Kolbenkörpers insbesondere durch Dosier-Rasthubstellungen über entsprechend ausgestaltete Rast- und/oder Anschlageinrichtungen vorgegeben werden.

Die Dosiervorrichtung nach diesem weiteren Aspekt kann eine Rasteinrichtung aufweisen, mit mindestens einem Rasterelement am Dosier-Kolbenkörper und mit mindestens einem Gegen-Rasterelement am Vorratsbehälter zur Vorgabe von mindestens zwei Dosier-Rasthubstellungen des Dosier-Kolbenkörpers im Vorratsbehälter, ausgehend von der Ausgangsposition, die entsprechende Dosierposition des Dosier-Kolbenkörpers vorgeben, die sich in der Wegstrecke zwischen der Ausgangsposition und der jeweiligen Dosierposition unterscheiden.

Dabei kann der Dosier-Kolbenkörper nach dem weiteren Aspekt eine äußere Dosierhülse und einen inneren Kolbenkörper aufweisen, der um eine Längsachse der Dosiervorrichtung in der Dosierhülse drehbar sein kann, wobei die Dosierhülse einen inneren Dosierhülsenabschnitt aufweist, der im Vorratsbehälter abgedichtet geführt ist, und einen äußeren Dosierhülsenabschnitt, der an einer Außenwand des Vorratsbehälters geführt ist, wobei die Rasteinrichtung im Bereich einer Führung des äußeren Dosierhülsenabschnitts am Vorratsbehälter angeordnet ist. Alternativ kann die Dosierhülse mit dem Kolbenkörper auch drehfest und insbesondere einstückig verbunden sein.

Die Dosiervorrichtung nach dem weiteren Aspekt kann eine Anschlageinrichtung mit einem axial am Vorratsbehälter festgelegten Anschlagskörper und einem axial an der Dosierhülse festgelegten Gegenanschlagkörper zur Vorgabe einer Dosier-Rasthubstellung des Dosier-Kolbenkörpers im Vorratsbehälter aufweisen, die eine entsprechende Dosierstellung des Dosier-Kolbenkörpers, ausgehend von einer eingeschobenen Ausgangsstellung des Dosier-Kolbenkörpers im Vorratsbehälter, darstellt.

Die Dosierhülse kann dabei einen inneren Dosierhülsenabschnitt aufweisen, der im Vorratsbehälter abgedichtet geführt ist, und einen äußeren Dosierhülsenabschnitt, der an einer Außenwand des Vorratsbehälters geführt ist, wobei der Anschlagkörper im Bereich einer Führung des äußeren Dosierhülsenabschnitts am Vorratsbehälter angeordnet ist.

Der Anschlagkörper kann bei diesem weiteren Aspekt in einem Langloch der Dosierhülse zur Vorgabe der Dosierstellung verschiebbar sein.

Bei diesem weiteren Aspekt der Dosiervorrichtung kann der Anschlagkörper eine Verriegelungsstruktur aufweisen und verlagerbar sein zwischen einer Freigabestellung, in der die Verriegelungsstruktur wirkungslos ist, und einer Verriegelungsstellung, in der die Verriegelungsstruktur mit einer komplementären Verriegelungs-Gegenstruktur zusammenwirkt, die am Vorratsbehälter ausgeführt ist, sodass in der Verriegelungsstellung der Anschlagkörper axial am Vorratsbehälter festgelegt ist.

Dabei kann eine erste Rasteinrichtung den Anschlagkörper überwindbar in der Freigabestellung sichern und eine zweite Rasteinrichtung kann den Anschlagkörper nach Überwindung der ersten Rasteinrichtung in der Verriegelungsstellung sichern.

Auch bei dem weiteren Aspekt der Dosiervorrichtung kann der Vorratsbehälter zweiteilig sein und einen inneren Dosierzylinder mit einem Vorratsvolumen und eine äußere Führungshülse aufweisen, wobei der Dosierzylinder und die Führungshülse axial und, in einer axialen Relativ-Endstellung, gegen eine Relativdrehung zueinander um die Längsachse aneinander gesichert sind.

Dabei kann eine Axial-Drehsicherungseinrichtung vorgesehen sein, die in einer ersten Axialposition des Dosierzylinders zur Führungshülse eine Relativdrehung des Dosierzylinders zur Führungshülse um die Längsachse zulässt und in einer zweiten Axialposition des Dosierzylinders zur Führungshülse eine Relativdrehung des Dosierzylinders zur Führungshülse um die Längsachse sperrt, wobei über eine Relativdrehung des Dosierzylinders zur Führungshülse in der ersten Axialposition eine Feinvorgabe einer Axialposition des Anschlagkörpers zum Dosierzylinder und damit der Dosierposition des Dosier-Kolbenkörper im Vorratsbehälter, vorgebbar ist.

Die Vorteile einzelner Merkmale dieses weiteren Aspekts der Dosiervorrichtung entsprechen denen, die vorstehend im Zusammenhang mit der zuerst erläuterten erfindungsgemäßen Dosiervorrichtung bereits diskutiert wurden.

Ein Ausführungsbeispiel der Erfindung wird nachfolgend an Hand der Zeichnung näher erläutert. In dieser zeigen:
- Fig. 1 und 2: perspektivische Ansichten einer Dosiervorrichtung zur dosierten Verabreichung eines fließfähigen Präparates zusammen mit einem fließfähigen Trägermedium;
- Fig. 3: eine Ansicht der Dosiervorrichtung, die diese aufbauende Komponenten entlang einer Längsachse der Dosiervorrichtung gebrochen zeigt, so dass interne Details der Dosiervorrichtung sichtbar werden;
- Fig. 4: aus einer im Vergleich zu Fig. 3 anderen Perspektive, ansonsten ähnlich zu Fig. 3, einen Ausschnitt der Dosiervorrichtung im Bereich von Böden eines Vorratsbehälters, einer Dosierhülse und eines inneren Kolbenkörpers, die beide Komponenten eines Dosier-Kolbenkörpers darstellen;
- Fig. 5: perspektivisch eine weitere Ausführung einer Dosiervorrichtung zur dosierten Verabreichung eines fließfähigen Präparates zusammen mit einem fließfähigen Trägermedium;
- Fig. 6: perspektivisch und im Vergleich zur Fig. 5 vergrößert einen Anschlagkörper der Dosiervorrichtung nach Fig. 5;
- Fig. 7: perspektivisch einen inneren Dosierzylinder als Teil eines zweiteiligen Vorratsbehälters der Dosiervorrichtung nach Fig. 5;
- Fig. 8: perspektivisch eine äußere Führungshülse des zweiteiligen Vorratsbehälters der Dosiervorrichtung nach Fig. 5;
- Fig. 9: perspektivisch eine äußere Dosierhülse eines Dosier-Kolbenkörpers der Dosiervorrichtung nach Fig. 5;
- Fig. 10: perspektivisch einen inneren Kolbenkörper des Dosier-Kolbenkörpers der Dosiervorrichtung nach Fig. 5;
- Fig. 11: einen axialen Längsschnitt durch die Dosiervorrichtung nach Fig. 5;
- Fig. 12: in einer im Vergleich zur Fig. 11 um 180° um eine Hochachse gedrehten Ansicht vergrößert einen Ausschnitt im Verbindungsbereich zwischen der äußeren Führungshülse und dem inneren Dosierzylinder des Vorratsbehälters im Bereich einer formschlüssigen Verbindung dieser beiden Vorratsbehälter-Bauteile;
- Fig. 13: in einer vergrößerten und innere Details preisgebenden Detailansicht einen Verbindungsbereich zwischen der äußeren Führungshülse und der Dosierhülse der Ausführung nach Fig. 5 im Bereich einer Führung eines äußeren Dosierhülsenabschnitts an einer Außenwand der äußeren Führungshülse;
- Fig. 14: in einer perspektivischen Ausschnittsdarstellung der Ausführung nach Fig. 5 Details einer Drehführung der äußeren Dosierhülse am inneren Kolbenkörper;
- Fig. 15: ein Detail der Führung nach Fig. 14 im axialen Längsschnitt;
- Fig. 16: perspektivisch einen Querschnitt durch einen Verbindungsbereich der Dosiervorrichtung nach Fig. 5 auf Höhe des Anschlagkörpers, wobei der Anschlagkörper in einer Freigabestellung dargestellt ist;
- Fig. 17: in einer zu Fig. 16 ähnlichen perspektivischen Querschnittsdarstellung den Anschlagkörper in einer Verriegelungsstellung;
- Fig. 18: in einem axialen Querschnitt einen Ausschnitt der Dosiervorrichtung nach Fig. 5 im Bereich eines einem Mundstück gegenüberliegenden Dosierendes, mit dem Anschlagkörper in der Verriegelungsstellung;
- Fig. 19: in einer zu Fig. 5 ähnlichen perspektivischen Darstellung die Dosiervorrichtung nach Fig. 5 in einer Dosierstellung mit dem Anschlagkörper in der Verriegelungsstellung;
- Fig. 20: in einer zu Fig. 5 ähnlichen Darstellung eine weitere Ausführung einer Dosiervorrichtung zur dosierten Verabreichung eines fließfähigen Präparates zusammen mit einem fließfähigen Trägermedium;
- Fig. 21: in einer zu Fig. 6 ähnlichen Darstellung einen Anschlagkörper der Dosiervorrichtung nach Fig. 20;
- Fig. 22: in einer zu Fig. 7 ähnlichen Darstellung einen inneren Dosierzylinder als Teil eines Vorratsbehälters der Dosiervorrichtung nach Figur 20;
- Fig. 23: in einer zu Fig. 8 ähnlichen Darstellung eine äußere Führungshülse als weiteres Teil des Vorratsbehälters der Dosiervorrichtung nach Fig. 20;
- Fig. 24: in einer zu Fig. 9 ähnlichen Darstellung eine äußere Dosierhülse eines Dosier-Kolbenkörpers der Dosiervorrichtung nach Fig. 20;
- Fig. 25: in einer zu Fig. 10 ähnlichen Darstellung einen inneren Kolbenkörper des Dosier-Kolbenkörpers nach Fig. 20;
- Fig. 26: in einem axialen Längsschnitt durch die Dosiervorrichtung nach Fig. 20 mit dem Anschlagkörper in einer Freigabestellung;
- Fig. 27: eine Ausschnittsvergrößerung aus Fig. 26 im Verbindungsbereich des inneren Dosierzylinders zur Äußeren Führungshülse des Vorratsbehälters, wobei eine Axialsicherungseinrichtung in einer ersten Axialposition des Dosierzylinders zur Führungshülse dargestellt ist, in der eine Relativdrehung des Dosierzylinders zu Führungshülse zugelassen ist,
- Fig. 28: die Dosiervorrichtung nach Fig. 20 in einer zu Figur 18 ähnlichen Darstellung mit dem Anschlagkörper in der Verriegelungsstellung;
- Fig. 29: perspektivisch ein Dosierende der Dosiervorrichtung nach Fig. 20 mit dem Anschlagkörper in der Verriegelungsstellung und der Axialsicherungseinrichtung in der ersten Axialposition;
- Fig. 30: in einer zu Fig. 27 ähnlichen Darstellung die Axialsicherungseinrichtung in einer zweiten Axialposition des Dosierzylinders zu Führungshülse, bei der im Vergleich zur ersten Axialposition die Führungshülse in den Dosierzylinder eingeschoben ist und in der eine Relativdrehung des Dosierzylinders zur Führungshülse um eine Längsachse der Dosiervorrichtung gesperrt ist;
- Fig. 31: in einer zu Fig. 19 ähnlichen Darstellung die Dosiervorrichtung nach Fig. 20 in der Dosierstellung;
- Fig. 32: in einer zu Fig. 16 ähnlichen Darstellung den Anschlagkörper der Dosiervorrichtung nach Fig. 20 in der Freigabestellung; und
- Fig. 33: in einer zu Fig. 17 ähnlichen Darstellung den Anschlagkörper der Dosiervorrichtung nach Fig. 20 in der Verriegelungsstellung.

Eine Dosiervorrichtung 1 dient zur dosierten Verabreichung eines fließfähigen Präparates zusammen mit einem fließfähigen Trägermedium. Sämtliche Komponenten der Dosiervorrichtung 1 sind aus Kunststoff.

Die Dosiervorrichtung 1 hat eine im Wesentlichen zylindrische Grundform und hat einen im Wesentlichen zylindrischen Vorratsbehälter 2 für das Präparat, der über eine Vielzahl hexagonal rasterartig angeordneter Zugangsöffnungen 3 mit der Umgebung kommuniziert. Auch Ausführungen der Dosiervorrichtung 1 sind möglich, bei denen genau eine derartige Zugangsöffnung 3 oder auch eine andere Anzahl derartiger Zugangsöffnungen 3 vorhanden sind. Ein Dosier-Kolbenkörper 4 dient zur Dosierung des Präparates. Der Dosier-Kolbenkörper 4 hat eine äußere Dosierhülse 5 und einen inneren, hohlen Kolbenkörper 6 als Hauptkomponenten. Der innere Kolbenkörper 6 ist um eine Längsachse 7 der Dosiervorrichtung 1 in der Dosierhülse 5 geführt drehbar angeordnet. Die Drehführung hierfür ist gebildet durch einen Umfangsbund 8 des inneren Kolbenkörpers 6, der in einer komplementär hierzu ausgeführten Aufnahme 9 der Dosierhülse 5 gleiten kann.

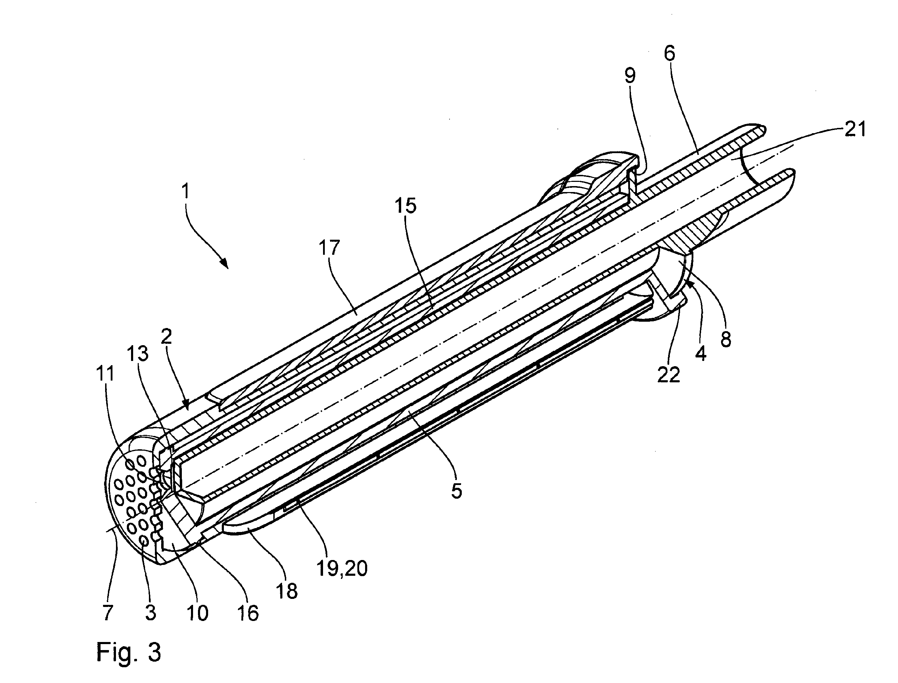

Die Dosierhülse 5 hat in einem Dosierhülsenboden 10, die den Zugangsöffnungen 3 des Vorratsbehälters 2 zugewandt ist, eine Dosierhülsenöffnung 11, die exzentrisch zur Längsachse 7 angeordnet ist. Die Dosierhülsenöffnung 11 ist vollständig außerhalb der zentralen Längsachse 7 angeordnet. Die Dosierhülsenöffnung 11 ist in der Fig. 3 gebrochen dargestellt und ist bei der ausgeführten Dosiervorrichtung 1 rund.

Der Dosierhülsenboden 10 ist gegen einen Boden 12 des inneren Kolbenkörpers 6 über einen Dichtkörper 13 abgedichtet, der gleichzeitig die Funktion eines Einwegeventils hat, wie nachfolgend noch erläutert wird. Der Dichtkörper / das Einwegeventil 13 ist in einer komplementär zu den Außenabmessungen des scheibenförmigen Dichtkörpers / Einwegeventils 13 gestalteten Ausnehmung 14a des Dosierhülsenbodens 10 angeordnet, die mit der Dosierhülsenöffnung 11 fluchtet.

Der innere Kolbenkörper 6 hat in den Zugangsöffnungen 3 des Vorratsbehälters 2 zugewandten Kolbenkörperboden 12 eine exzentrisch zur zentralen Längsachse 7 angeordnete Kolbenkörperöffnung 14. Auch die Kolbenkörperöffnung 14 des inneren Kolbenkörpers 6 ist vollständig außerhalb der zentralen Längsachse 7 angeordnet. Die Kolbenkörperöffnung 14 ist rund.

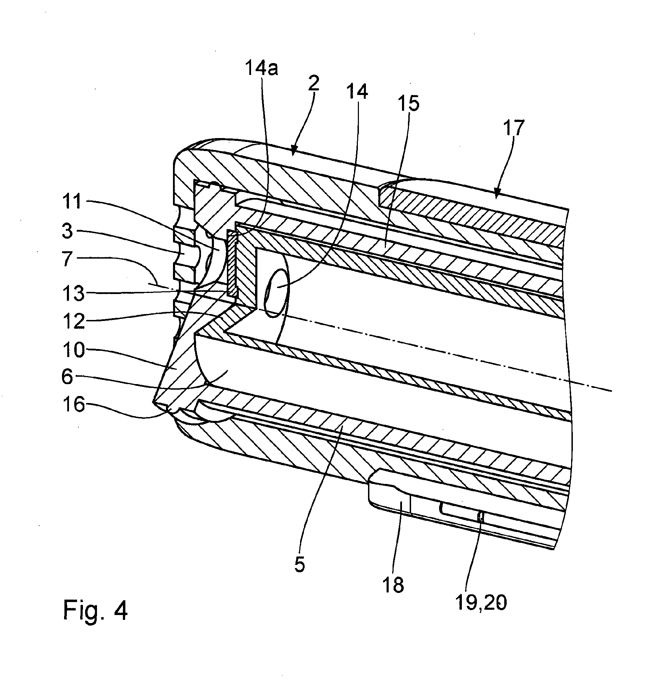

Die beiden Öffnungen 11, 14 der Dosierhülse 5 einerseits und des inneren Kolbenkörpers 6 andererseits sind in der Fig. 4 in einer relativen Drehposition um die zentrale Längsachse 7 der Dosierhülse 5 zum inneren Kolbenkörper 6 dargestellt, die zwischen einer Durchgangs-Relativ-Drehposition und einer Verschluss-Relativ-Drehposition der Dosierhülse 5 zum inneren Kolbenkörper 6 liegt. In der Durchgangs-Relativ-Drehposition der Dosierhülse 5 zum inneren Kolbenkörper 6 ist ein Durchgang für eine Mischung des fließfähigen Präparates und des fließfähigen Trägermediums zwischen dem Inneren des Vorratsbehälters 2 und dem Inneren des inneren Kolbenkörpers 6 durch die Dosierhülsenöffnung 11 und durch die Kolbenkörper 14 freigegeben. In der Durchgangs-Relativ-Drehposition fluchtet die Dosierhülsenöffnung 11 mit der Kolbenkörperöffnung 14. In dieser Durchgangs-Relativ-Drehposition wirkt der Dichtkörper / das Einwegeventil 13 so, dass ein Präparat- / Trägermediumfluss vom Vorratsbehälter 2 in den inneren Kolbenkörper 6 zugelassen ist und ein Präparat-/ Trägermediumfluss in Gegenrichtung gesperrt ist.

In der Verschluss-Relativ-Drehposition der Dosierhülse 5 zum inneren Kolbenkörper 6 ist ein Durchgang zwischen dem Inneren des Vorratsbehälters 2 und dem Inneren des inneren Kolbenkörpers 6 verschlossen. In dieser Stellung wirkt der Dichtkörper / das Einwegeventil 13 vollständig zwischen den beiden Böden 10, 12 dichtend, so dass kein Präparat- / Trägermediumfluss zwischen dem Vorratsbehälter 2 und dem Inneren des inneren Kolbenkörpers 6 möglich ist.

Die Dosierhülse 5 hat einen inneren Dosierhülsenabschnitt 15. In diesem ist der innere Kolbenkörper 6 zwischen dem Umfangsbund 8 und dem Kolbenkörperboden 12 angeordnet. Der innere Dosierhülsenabschnitt 15 ist im Vorratsbehälter 2 abgedichtet geführt. Hierzu dient eine um den Dosierhülsenboden 10 mantelseitig umlaufende und an diesem angeformte Dichtung 16, die als Lippendichtung ausgeführt ist.

Einstückig angeformt ist an den inneren Dosierhülsenabschnitt 15 ein äußerer Dosierhülsenabschnitt 17, der durch zwei gegenüberliegende Führungsnasen 18 gebildet ist, die an einer Außenwand des Vorratsbehälters geführt sind. Im Bereich einer Führung des äußeren Dosierhülsenabschnitts 17, also der beiden Führungsnasen 18, am Vorratsbehälter 2 ist eine Rasteinrichtung 19 angeordnet. Die Rasteinrichtung 19 hat als Kerben ausgestaltete Rastelemente 20 (vgl. Fig. 3) an den einander gegenüberliegenden und längs der Längsachse 7 verlaufenden Längsseiten der Führungsnasen 18 und ein in der Zeichnung nicht dargestelltes Gegen-Rastelement am Vorratsbehälter 2, das als den Kerben 20 gegenüberliegende Rastrippe in den Außenwandführungen des Vorratsbehälters 2 eingeformt ist. Die Rasteinrichtung 19 dient zur Vorgabe von mindestens zwei Dosier-Rasthubstellungen des Dosier-Kolbenkörpers 4.

Über die Dichtung 16 gegen eine Innenwand des Vorratsbehälters 2 abdichtet, ist der innere Dosierhülsenabschnitt 15 der Dosierhülse 5 des Dosier-Kolbenkörpers 4 in dem Vorratsbehälters 2 abgedichtet verlagerbar zwischen einer in der Zeichnung dargestellten Ausgangsposition, in der der Dosier-Kolbenkörper 4 ganz in dem Vorratsbehälter 2 eingeschoben ist, und den verschiedenen, durch die Rasteinrichtung 19 vorgegebenen Dosierpositionen, in der der Dosier-Kolbenkörper 4 bis zu einer der jeweils gewünschten Dosiermenge des Präparats entsprechende Wegstrecke aus dem Vorratsbehälter 2 herausgezogen ist.

Die jeweilige Dosierposition ist dann erreicht, wenn die von der Rasteinrichtung vorgegebene Dosier-Rasthubstellung erreicht ist. Die Rasteinrichtung 19 dient also zur Vorgabe der durch die Rastelemente 20 definierten Dosier-Rasthubstellungen des Dosier-Kolbenkörpers 4, ausgehend von der Ausgangsposition, die sich in der Wegstrecke zwischen der Ausgangsposition und der jeweiligen Dosierposition unterscheiden.

Die Dosiervorrichtung 1 wird folgendermaßen verwendet: Zunächst wird eine gewünschte Dosiermenge des Präparats in den Vorratsbehälter 2 der Dosiervorrichtung 1 über den Dosier-Kolbenkörpers 4 eingesogen. Hierzu wird die Dosiervorrichtung 1 mit dem die Zugangsöffnungen 3 aufweisenden Ende in das fließfähige Präparat eingetaucht. Anschließend wird der Dosier-Kolbenkörper 4 ausgehend von der Ausgangsposition in die gewünschte Dosier-Position aufgezogen, die mittels der Rasteinrichtung 19 durch eine entsprechende Anzahl von Klicks, die durch das Vorbeigleiten der Rastelemente 20 am Gegen-Rastelement erzeugt werden, signalisiert wird.

Dieses Aufziehen findet in der Verschluss-Relativ-Drehposition der Dosierhülse 5 zum inneren Kolbenkörper 6 statt.

Wenn das Präparat eindosiert ist, kann die so vorbereitete Dosiervorrichtung 1 in einen Behälter mit dem Trägermedium eingetaucht werden.

Nun kann ein Benutzer an einer dem Kolbenkörperboden 2 gegenüberliegenden, von außen zugänglichen Ansaugöffnung 21 des inneren Kolbenkörpers 6 ansaugen. Vorher verdreht der Benutzer den inneren Kolbenkörper 6 relativ zur Dosierhülse 5 in die Durchgangs-Relativ-Drehposition. Beim Ansaugen wird einerseits das im Vorratsbehälter 2 vorliegende Präparat und wird andererseits das Trägermedium durch die Zugangsöffnungen 3, die Dosierhülsenöffnung 11, den Dichtkörper / das Einwegeventil 13 und die Kolbenkörperöffnung 14 in das Innere des inneren Kolbenkörpers 6 eingesogen. Im inneren Kolbenkörper 6 findet nun beim Fluss des Präparates und des Trägermediums hin zur Ansaugöffnung 21 eine Vermischung des Präparates mit dem Trägermedium statt. Diese Mischung wird dann vom Benutzer wie durch einen Strohhalm eingesogen.

Eine Relativ-Drehposition des inneren Kolbenkörpers 6 zur äußeren Dosierhülse 5 kann durch entsprechende Text- oder Symbolmarkierungen auf einander benachbarten Sichtflächen dieser beiden Komponenten angezeigt sein. Diese Relativ-Drehpositionsanzeige kann alternativ oder zusätzlich durch eine Dreh-Rastung geschehen, die dem Benutzer die jeweilige Relativ-Drehposition haptisch anzeigt. Zur Einstellung einer benötigten Dosiermenge der Dosiervorrichtung 1 weist diese im Bereich der Führung der Führungsnasen 18 im Vorratsbehälter 2 eine Skala, beispielsweise eine Volumenskala, auf.

Der innere Dosierhülsenabschnitt 15 und der äußere Dosierhülsenabschnitt 17 sind miteinander über einen Stegabschnitt 22 verbunden, der gleichzeitig die Aufnahme 9 für den Umfangsbund 8 des inneren Kolbenkörpers 6 begrenzt. Insgesamt hat der Stegabschnitt 22 zwei Stege zur Verbindung des inneren Dosierhülsenabschnitts 15 mit den beiden Führungsnasen 18.

Bei einer alternativen Ausführung kann der Dichtkörper 13 auch so angeordnet sein, dass er die Dosierhülsenöffnung 11 nicht abdeckt, sondern diese beispielsweise in der Form eines O-Rings umgibt, der den Dosierhülsenboden 10 gegen den Kolbenkörperboden 12 des inneren Kolbenkörpers 6 abdichtet. In diesem Fall entfällt die Ventilfunktion des Dichtkörpers 13. Der Dichtkörper / das Einwegeventil 13 kann auch drehfest mit dem inneren Kolbenkörper 6, zum Beispiel in einer Ausnehmung von diesem, angeordnet sein.

Mindestens eine der beiden Öffnungen 11, 14 kann auch als in Umfangsrichtung gebogen verlaufendes Langloch ausgeführt sein.

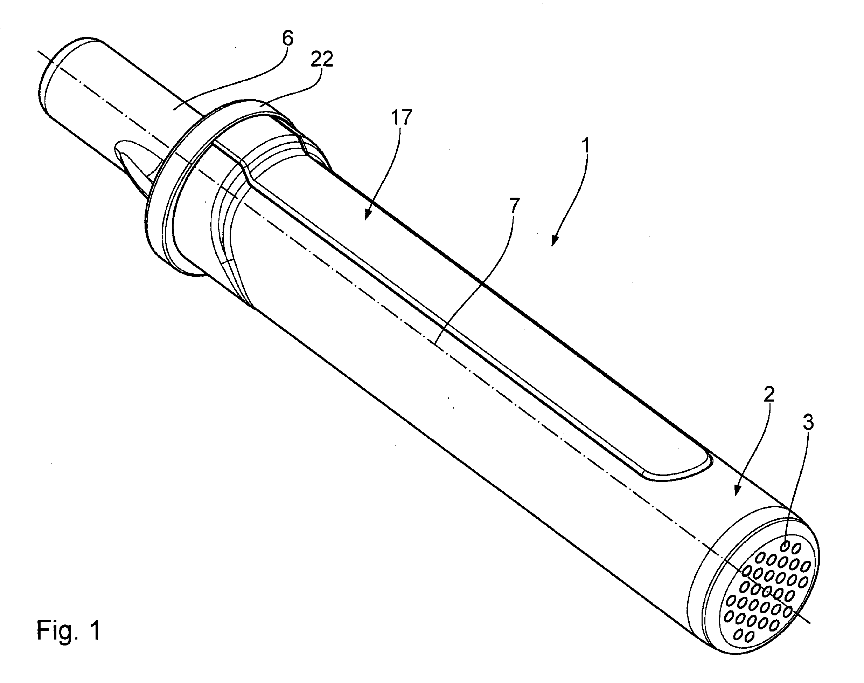

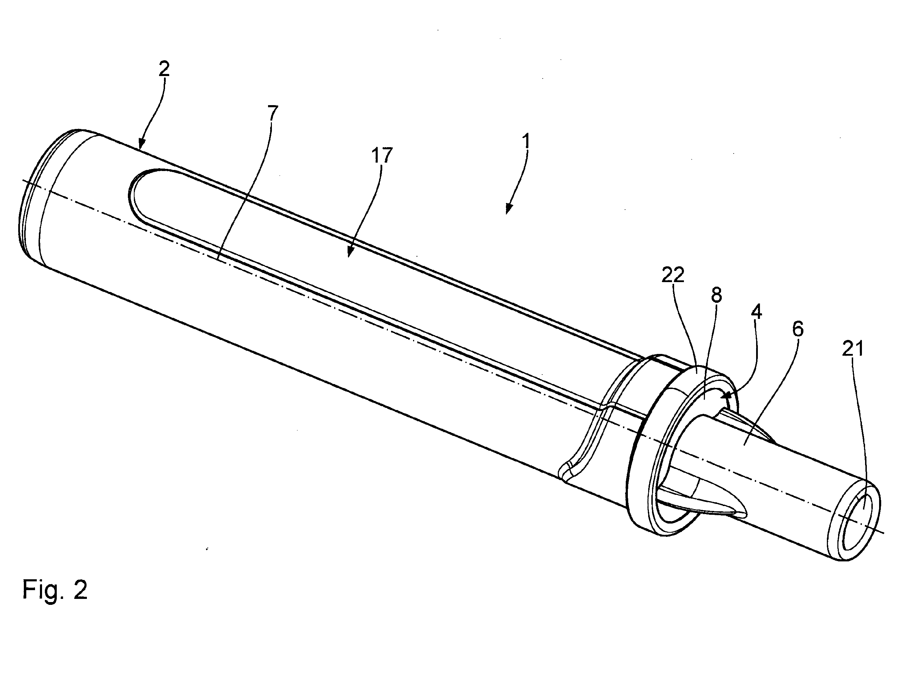

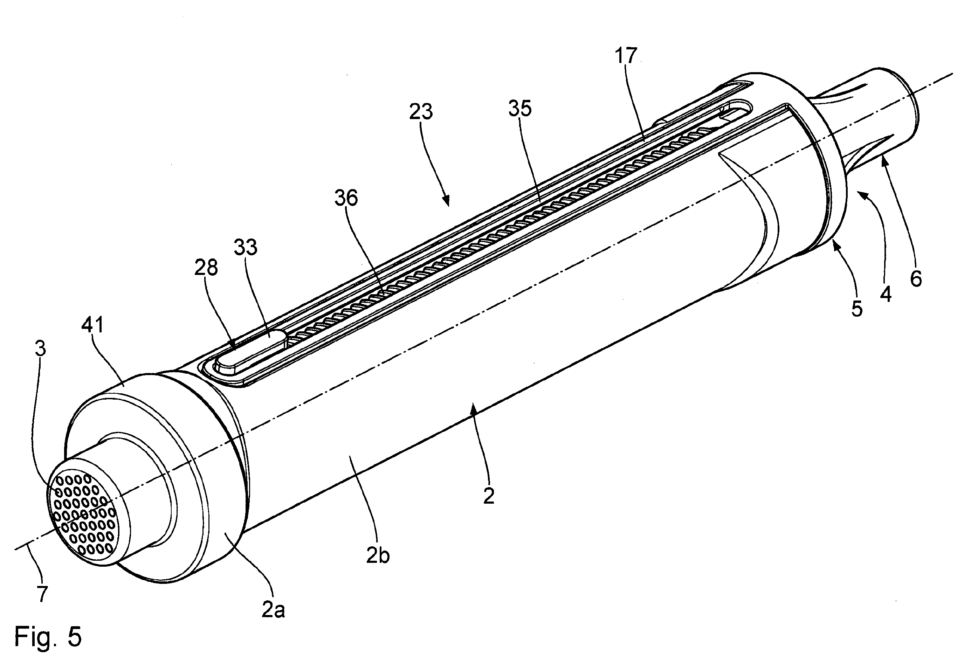

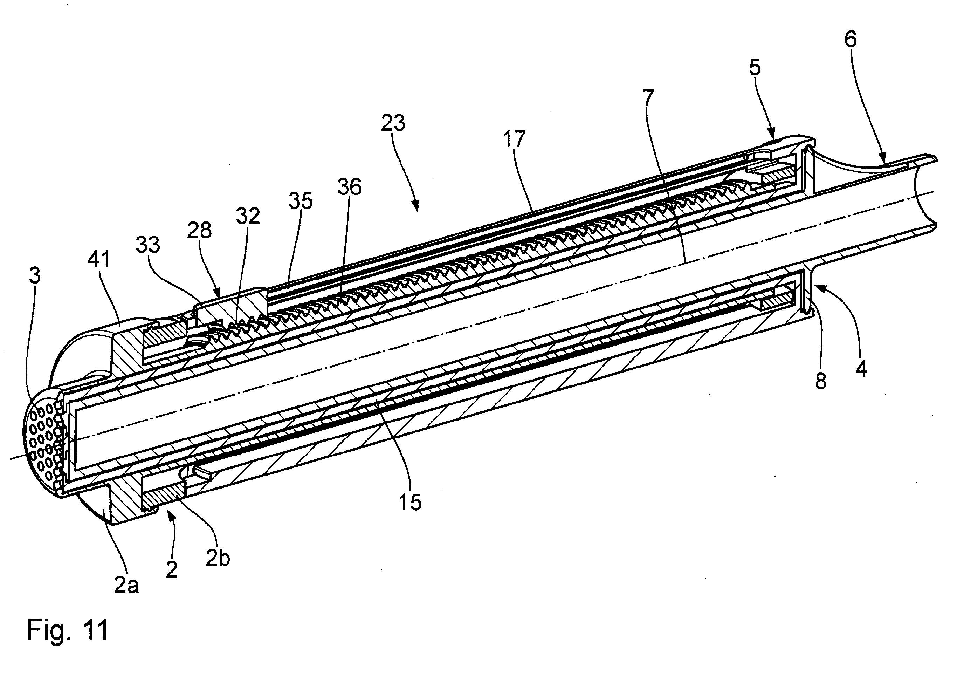

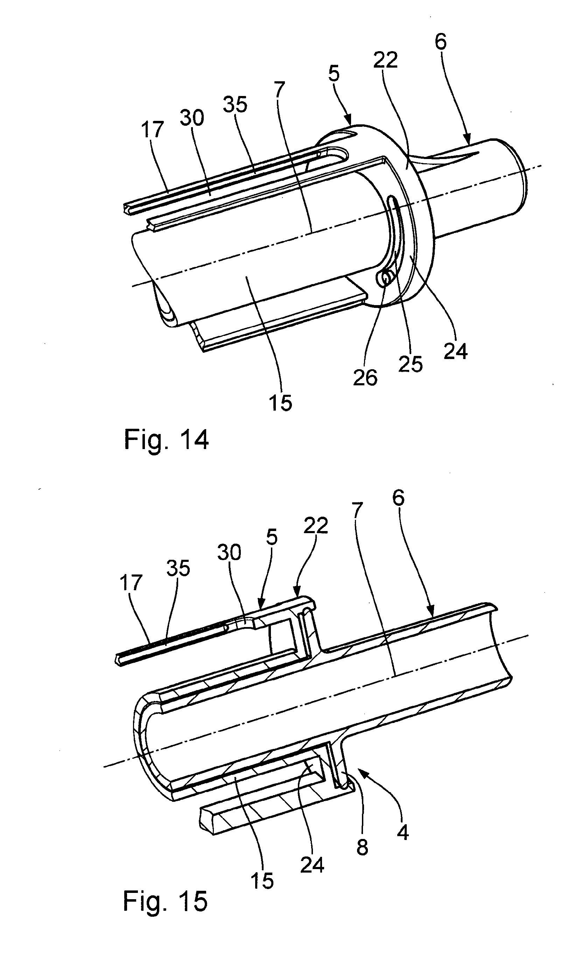

Anhand der Fig. 5 bis 19 wird nachfolgend eine weitere Ausführung einer Dosiervorrichtung 23 beschrieben. Komponenten, die in denjenigen entsprechen, die vorstehend oder in Bezugnahme auf die Dosiervorrichtung 1 nach den Fig. 1 bis 4 bereits erläutert wurden, tragen die gleichen Bezugsziffern und werden nicht nochmals im Einzelnen diskutiert.

Auch bei der Dosiervorrichtung 23 ist der Dosier-Kolbenkörper 4 zweiteilig mit der äußeren Dosierhülse 5 und dem inneren Kolbenkörper 6.

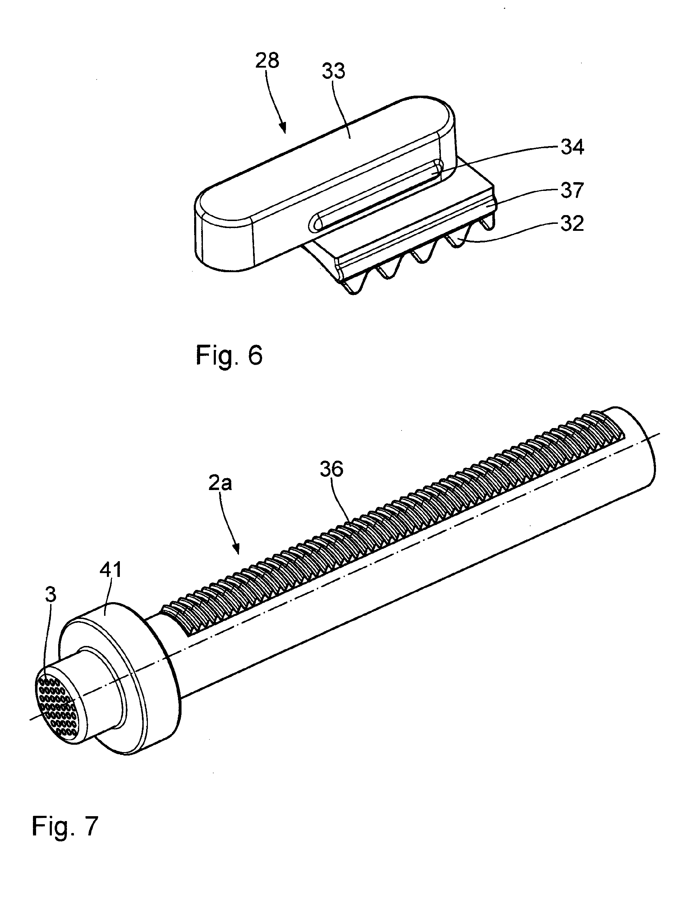

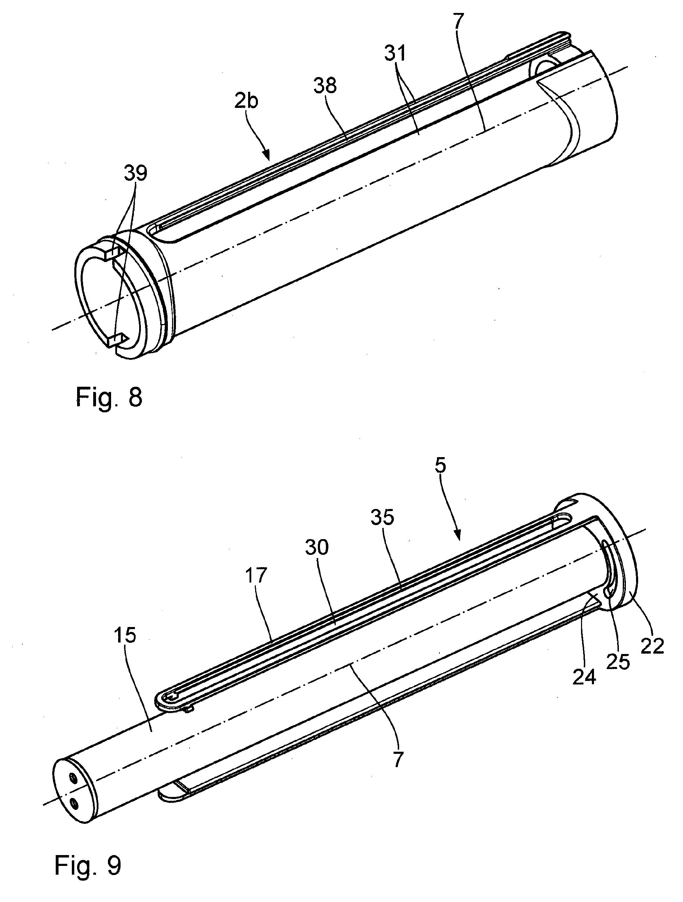

Bei der Dosiervorrichtung 23 ist der Vorratsbehälter 2 zweiteilig ausgeführt und umfasst einen inneren Dosierzylinder 2a, der perspektivisch in der Fig. 7 dargestellt ist und eine äußere Führungshülse 2b, die perspektivisch in der Fig. 8 dargestellt ist. Im inneren Dosierzylinder 2a ist das Vorratsvolumen des Vorratsbehälters 2 für das fließfähige Präparat ausgebildet.

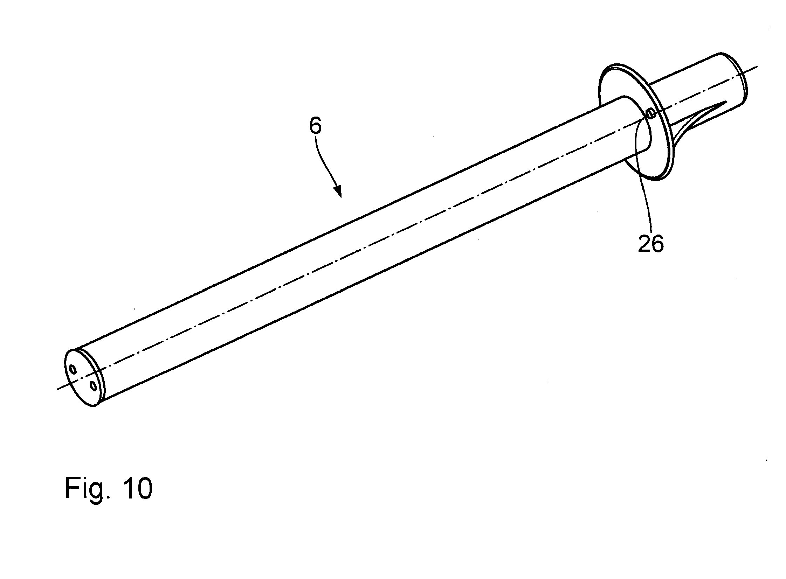

Bei der Dosiervorrichtung 23 ist eine Führung einer Drehbewegung der äußeren Dosierhülse 5, die perspektivisch in der Fig. 9 dargestellt ist, zum inneren Kolbenkörper 6, der perspektivisch in der Fig. 10 dargestellt ist, durch eine Langloch-Stiftführung gestaltet. Diese umfasst ein teilkreisförmiges und in Umfangsrichtung um die Längsachse 7 der Dosiervorrichtung 23 in einer mundstückseitigen Ring-Grundplatte 24 des Stegabschnitts 22 der Dosierhülse 5 angeordnetes Langloch 25 und einen in dem Langloch 25 laufenden Führungsstift 26, der am inneren Kolbenkörper 6 angeformt ist. Der Führungsstift 26 erstreckt sich axial beabstandet zur Längsachse 7 und parallel zu dieser. Auch eine umgekehrte Gestaltung, bei der das Langloch im Kolbenkörper 6 und der Führungsstift in der Dosierhülse 5 ausgeführt ist, ist möglich.

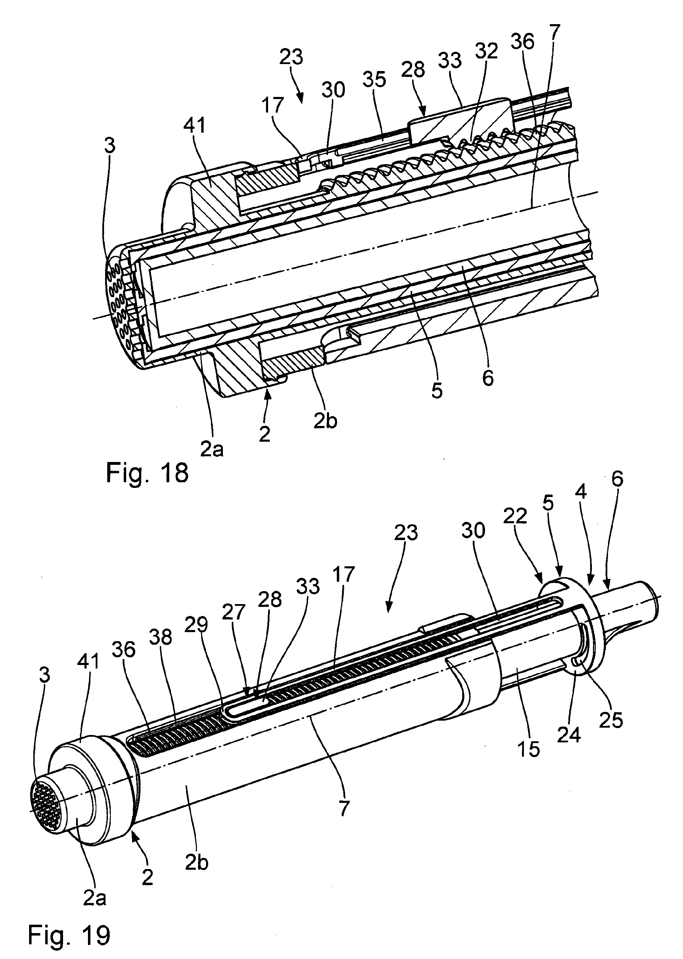

Zur Begrenzung einer Dosierstellung der Dosiereinrichtung 23, die in der Fig. 19 dargstellt ist, hat die Dosiervorrichtung 23 eine Anschlageinrichtung 27. Diese umfasst einen axial am Vorratsbehälter 2 festgelegten Anschlagkörper 28, der perspektivisch in der Fig. 6 dargstellt ist, und einen axial am Dosier-Kolbenkörper 4 festgelegten Gegenanschlagkörper 29 in Form eines Langlochs 30 eines äußeren Dosierhülsenabschnitts 17 der Dosierhülse 5.

Die Anschlageinrichtung 27 dient zur Vorgabe einer bestimmten Dosier-Rasthubstellung aus einer Mehrzahl auswählbarer Dosier-Rasthubstellungen des Dosier-Kolbenkörpers 4 im Vorratsbehälter 2 der Dosiervorrichtung 1. Über die Anschlageinrichtung 27 ist also eine jeweilige Dosierstellung des Dosier-Kolbenkörpers 4 relativ zum Vorratsbehälter 2, z.B. die Dosierstellung nach Fig. 19, ausgehend von einer eingeschobenen Ausgangsstellung des Dosier-Kolbenkörpers 4 im Vorratsbehälter 2 vorgebbar.

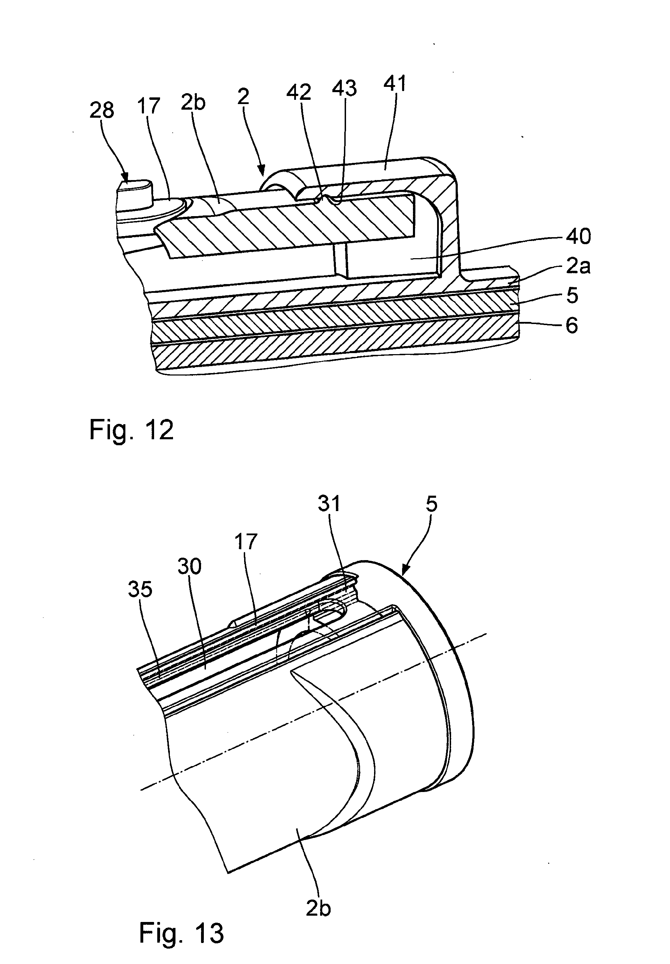

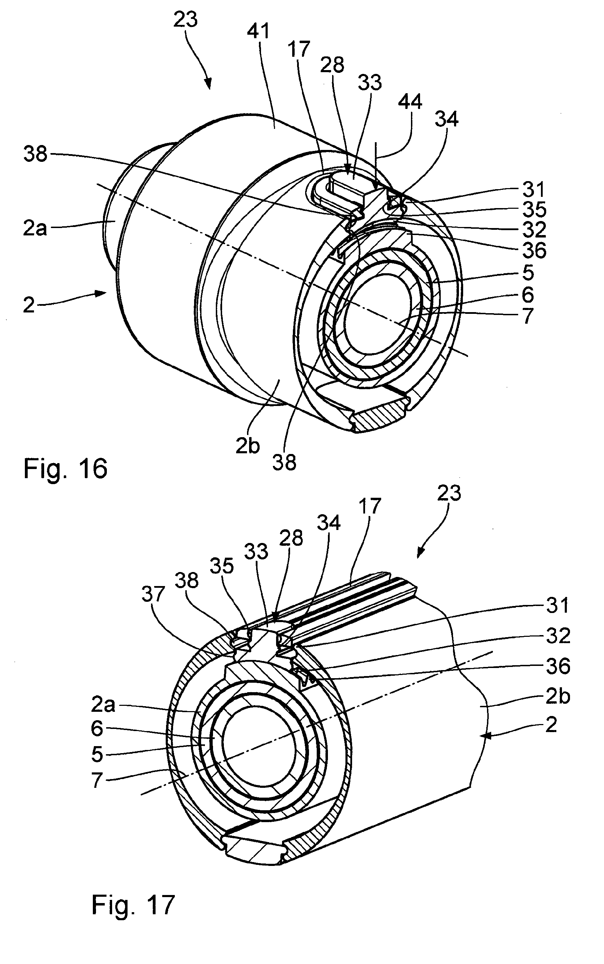

Die Dosierhülse 5 der Dosiervorrichtung 23 hat entsprechend der Dosiervorrichtung 1 neben dem äußeren Dosierhülsenabschnitt 17 noch den inneren Dosierhülsenabschnitt 15. Der innere Dosierhülsenabschnitt 15 ist im Vorratsbehälter 2 abgedichtet geführt. Der äußere Dosierhülsenabschnitt 17 ist, wie im Detail in der Fig. 13 dargestellt, an einer Außenwand 31 des Vorratsbehälters 2 geführt. Der Anschlagkörper 28 ist, wie in den Querschnittsdarstellungen nach Fig. 16 und 17 verdeutlicht, im Bereich der Führung des äußeren Dosierhülsenabschnitts 17 an der Außenwand 31 des Vorratsbehälters 2 angeordnet.

Zur Vorgabe der Dosierstellung ist der Anschlagkörper 28 im Axialverlauf in dem Langloch 30 der Dosierhülse 5 verschiebbar. Auch beim Dosieren der Dosiervorrichtung 23 verschiebt sich der Anschlagkörper 28 relativ zum Langloch 30.

Bei der Vorgabe der Dosierstellung liegt der Anschlagkörper 28 in einer Freigabestellung vor, die in der Fig. 16 dargestellt ist. In der Freigabestellung ist eine sägezahnartige Verriegelungsstruktur 32 des Anschlagkörpers 28 wirkungslos. Die Verriegelungsstruktur 32 ist gegenüberliegend einer von außen zugänglichen Betätigungswand 33 des Anschlagkörpers 28 ausgebildet und hat einen quer zur Längsachse 7 verlaufenden Zahnverlauf. In der Freigabestellung des Anschlagkörpers 28 werden Axialrippen 34 des Anschlagkörpers 28 in komplementär hierzu geformten Axialnuten 35 des äußeren Dosierhülsenabschnitts 17 geführt.

Ausgehend von der Freigabestellung ist der Anschlagkörper 28 radial zur Längsachse 7 in eine Verriegelungsstellung verlagerbar, die in der Fig. 17 im Detail dargestellt ist. In der Verriegelungsstellung wirkt die Verriegelungsstruktur 32 des Anschlagkörpers 28 mit einer komplementären Verriegelungs-Gegenstruktur 36 am inneren Dosierzylinder 2a zusammen, die bei der Dosiervorrichtung 23 als Zahnleiste ausgeführt ist.

In der Verriegelungsstellung des Anschlagkörpers 28 ist der Anschlagkörper 28 axial am Vorratsbehälter 2 festgelegt. In der Verriegelungsstellung hintergreifen weitere Axialrippen 37 des Anschlagkörpers 28 randseitige Wandbegrenzungen 38 der äußeren Führungshülse 2b, die dem Anschlagkörper 28 zugewandt sind.

Der Anschlagkörper 28 verbreitert sich, ausgehend von einer von außen zugänglichen Betätigungsseite der Betätigungswand 33 hin zur Verriegelungsstruktur 32 über eine Stufe, sodass eine Umfangserstreckung des Anschlagkörpers um die Längsachse 7 im Bereich der Verriegelungsstruktur 32 einerseits nur geringfügig kleiner ist als die Umfangserstreckung der Zahnleiste 36 und andererseits in etwa der Umfangserstreckung des äußeren Dosierhülsenabschnitts 17 entspricht.

Die Axialrippen 34 und die Axialnuten 35 stellen eine erste Rasteinrichtung dar, die den Anschlagkörper 28 überwindbar in der Freigabestellung sichert.

Die Axialrippen 37 und die Wandbegrenzungen 38 stellen eine zweite Rasteinrichtung dar, die den Anschlagkörper 28 nach Überwindung der ersten Rasteinrichtung 34, 35 in der Verriegelungsstellung sichert.

Der innere Dosierzylinder 2a und die Führungshülse 2b sind in einer axialen Relativ-Endstellung, die im Detail aus der Fig. 12 ersichtlich ist, gegen eine Relativdrehung zueinander um die Längsachse 7 aneinander gesichert. Zusätzlich sind der innere Dosierzylinder 2a und die äußere Führungshülse 2b relativ zueinander axial gesichert.

Die Drehsicherung ist gegeben durch zwei Radialnuten 39 (vgl. Fig. 8), die an den Zugangsöffnungen 3 zugewandten Ende in der äußeren Führungshülse 2b ausgeführt sind und in komplementär hierzu ausgeformte Radialrippen 40 eingreifen, die am inneren Dosierzylinder 2a im Bereich einer die Führungshülse 2b endseitig übergreifenden Ringkappe 41 angeformt sind. Die Axialsicherung des inneren Dosierzylinders 2a an der äußeren Führungshülse 2b ist gegeben durch eine äußere Umfangsrippe 42, die an der äußeren Führungshülse 2b angeformt ist und in eine komplementär hierzu ausgeführte innere Umfangsnut 43 eingreift.

Bei einer alternativen, nicht dargestellten Ausführung ist die Radialrippe 40 an der Führungshülse 2b und die Radialnut 39 im Dosierzylinder 2a ausgeführt.

Die Dosiervorrichtung 23 wird folgendermaßen verwendet: Zunächst wird über die Anschlageinrichtung 27 die gewünschte Dosiermenge, das heißt, die über den Anschlag erreichbare Dosierstellung, vorgegeben. Hierzu wird der Anschlagkörper 28 in der Freigabestellung so weit axial relativ zum inneren Dosierzylinder 2a und dessen Verriegelungs-Gegenstruktur 36 axial verlagert, bis der gewünschte Dosierhub erreicht ist. Anschließend wird eine radiale Kraft auf die Betätigungswand 33 des Anschlagkörpers 28 ausgeübt (vgl. Kraftpfeil 44 in der Fig. 16). Der Anschlagkörper überwindet dabei die erste Rasteinrichtung 34, 35 und rastet in die Verriegelungsstellung ein, die durch die zweite Rasteinrichtung 37, 38 gesichert wird. Nun wird, ausgehend von dem vollständig in den Vorratsbehälter 2 eingeschobenen Dosier-Kolbenkörper 4, die gewünschte Dosiermenge des Präparates in den Vorratsbehälter 2 eingesogen, wie dies vorstehend im Zusammenhang mit der Dosiervorrichtung 1 bereits erläutert wurde. Das Aufziehen des Dosier-Kolbenkörpers 4 geschieht, bis der Anschlag durch die Anschlageinrichtung 27 erreicht ist, bis also der Anschlagkörper 28 mit dem Gegenanschlagkörper 29, also dem Ende des Langlochs 30, zusammen wirkt. Wenn das Präparat eindosiert ist, kann die so vorbereitete Dosiervorrichtung 23 in dem Behälter mit dem Trägermedium eingetaucht und verabreicht werden, wie vorstehend im Zusammenhang mit der Dosiervorrichtung 1 bereits erläutert. Alternativ kann die Dosiervorrichtung 23 nach dem Eindosieren des Präparates auch wie eine Spritze verwendet werden. Das eindosierte Präparat kann also wie bei einer Spritze durch Einschieben des Dosier-Kolbenkörpers 4 aus dem Vorratsvolumen des Vorratsbehälters 2 wieder ausgedrückt werden.

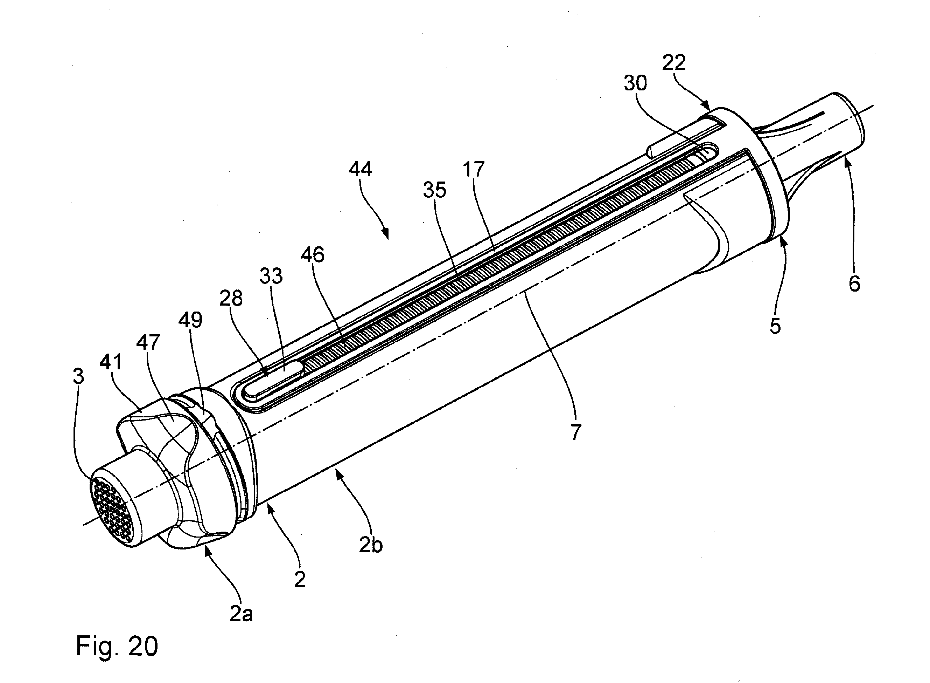

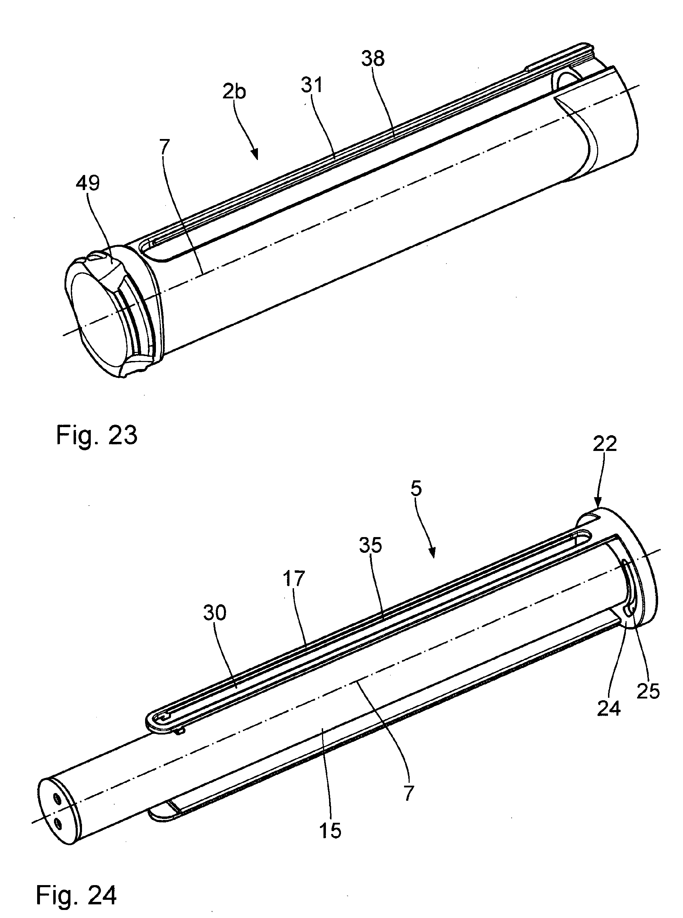

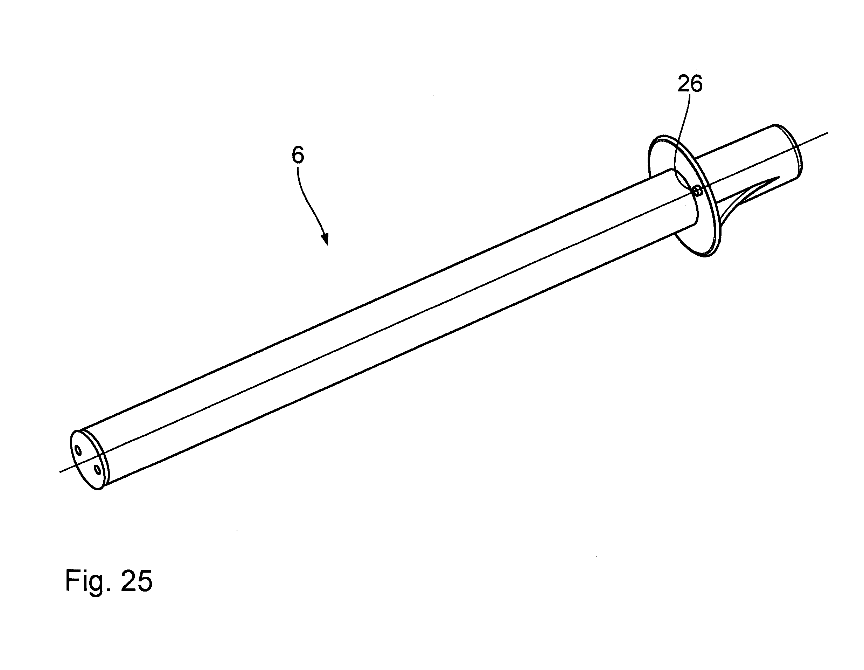

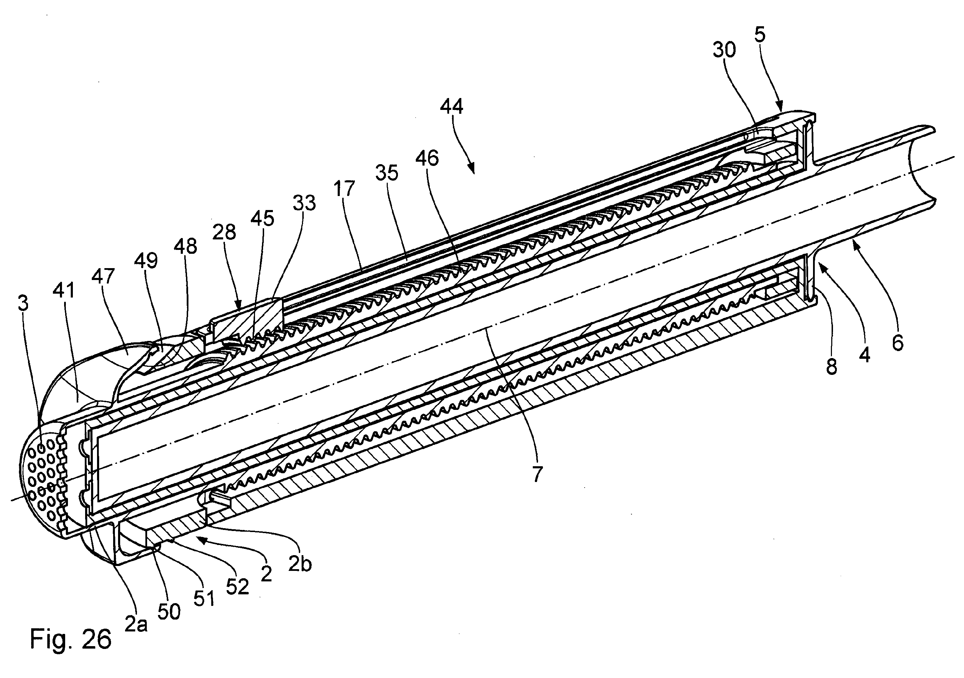

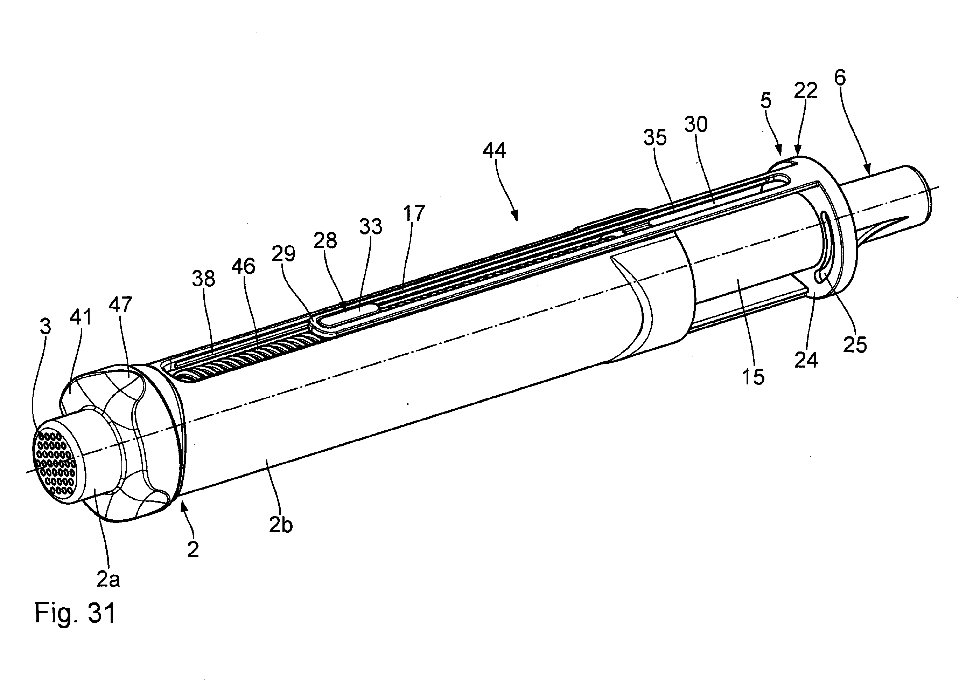

Anhand der Fig. 20 bis 33 wird nachfolgend eine weitere Ausführung einer Dosiervorrichtung 44 erläutert. Komponenten, die denjenigen entsprechen, die vorstehend unter Bezugnahme auf die Dosiervorrichtungen 1 und 23 bereits erläutert wurden, tragen die gleichen Bezugsziffern und werden nicht nochmals im Einzelnen diskutiert.

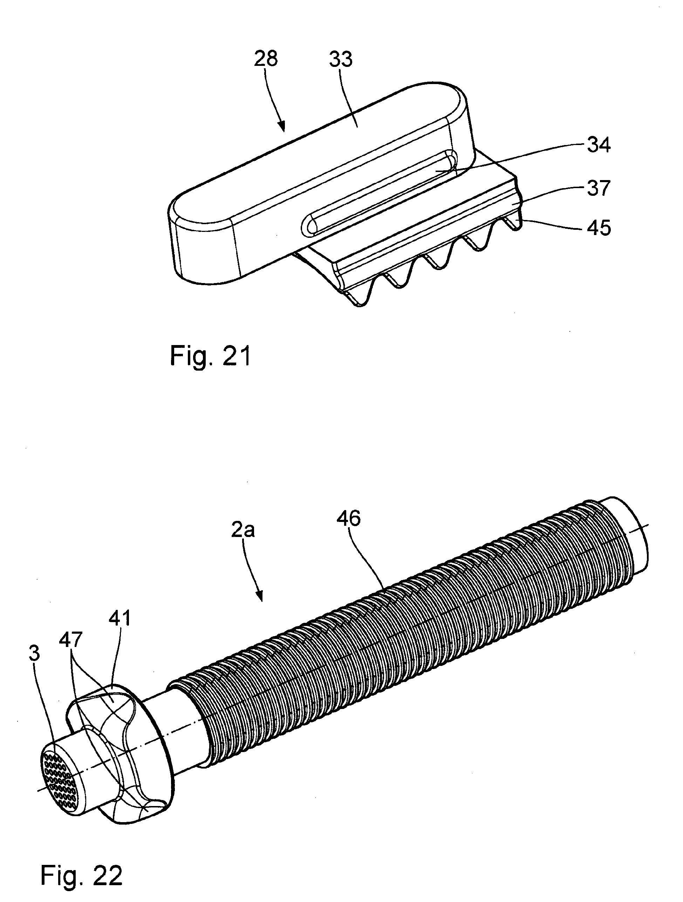

Der Anschlagkörper 28 (vgl. Fig. 21) der Dosiervorrichtung 44 hat eine Verriegelungsstruktur 45, die komplementär zu einer als Gewinde ausgeführten Verriegelungs-Gegenstruktur 46 des inneren Dosierzylinders 2a der Dosiervorrichtung 44 ausgeführt ist.

Eine Drehsicherung gegen eine Relativdrehung des inneren Dosierzylinders 2a zur äußeren Führungshülse 2b ist bei der Dosiervorrichtung 44 durch nicht rotationssymmetrisch gestaltete und zueinander komplementäre Umfangs- bzw. Stirnwände des inneren Dosierzylinders 2a und der äußeren Führungshülse 2b gestaltet. Hierzu hat die Ringkappe 41 des inneren Dosierzylinders 2a in Umfangsrichtung mehrere Einbuchtungen 47, die sich in einer Innenwand der Ringkappe 41 des inneren Dosierzylinders 2a hin zu einem stirnseitigen Endbereich der Führungshülse 2b fortsetzen. Hierdurch ergeben sich innere Drehsicherungs-Wandabschnitte 48 der Ringkappe 41 des inneren Dosierzylinders 2a. Hierzu komplementär hat der stirnseitige Endbereich der Führungshülse 2b äußere Drehsicherungs-Wandabschnitte 49.

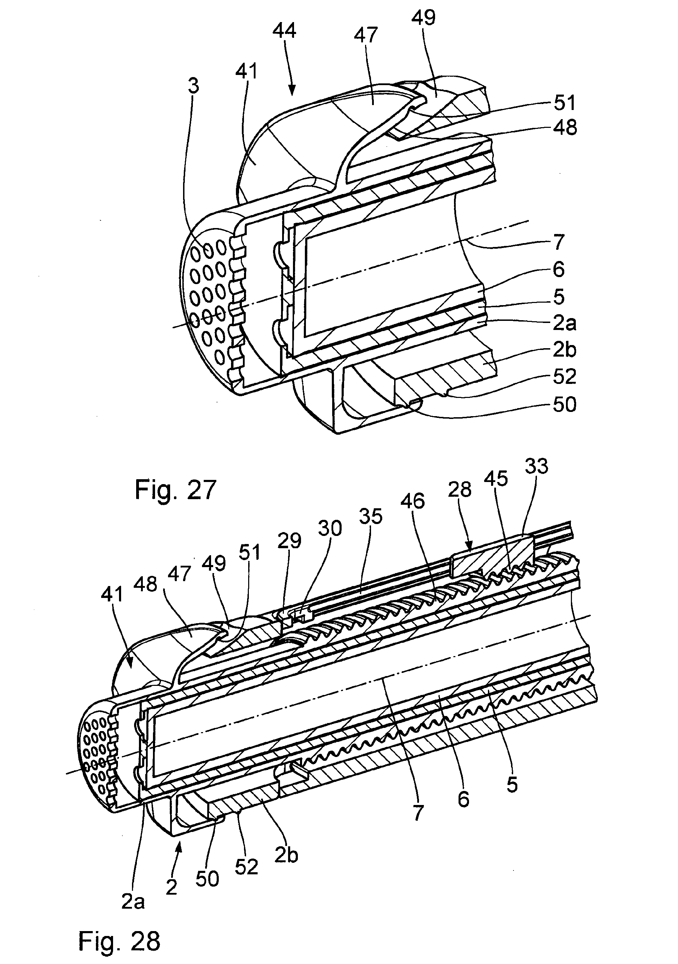

Fig. 27 zeigt die Führungshülse 2b in einer Axialposition relativ zum Dosierzylinder 2a. In dieser kommen die beiden Drehsicherungs-Wandabschnitte 48, 49 ausreichend voneinander frei, sodass eine Relativverdrehung des Dosierzylinders 2a relativ zur Führungshülse 2b um die Längsachse 7 möglich ist. Die Axialposition nach Fig. 27 ist axial gesichert über eine Umfangsrippe 50, die am stirnseitigen Endbereich der Führungshülse 2b angeformt ist und die in eine hierzu komplementär ausgeführte Umfangsnut 51 im freien Endabschnitt der Ringkappe 41 des Dosierzylinders 2a eingreift. In der Freigabe-Axialposition nach Fig. 27 ist eine Relativdrehung des Dosierzylinders 2a zur Führungshülse 2b möglich.

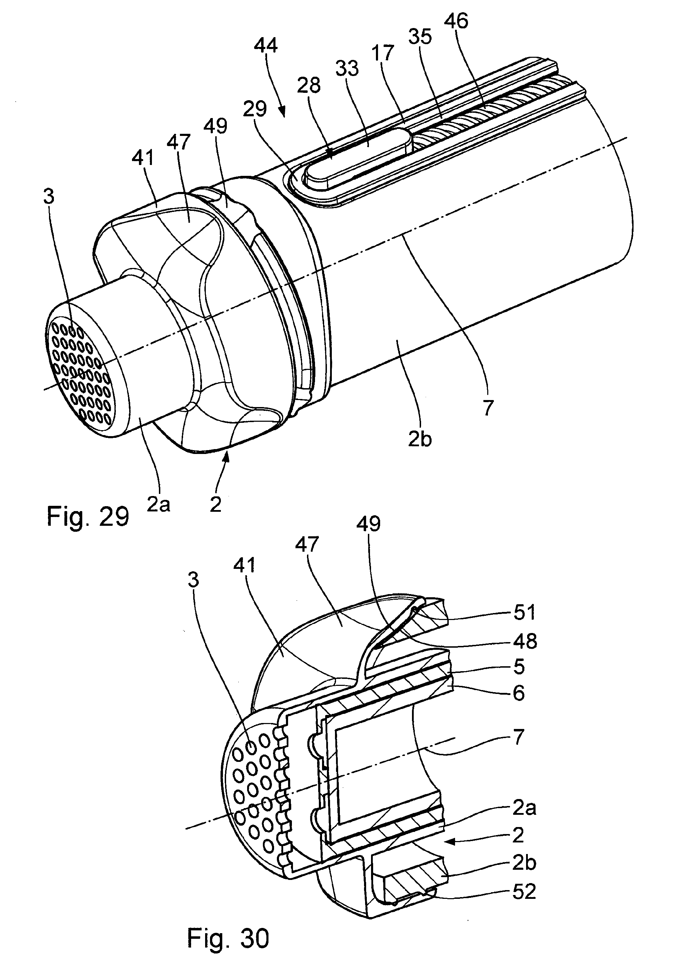

Fig. 30 zeigt eine weitere Axialposition des Dosierzylinders 2a relativ zur Führungshülse 2b, die, ausgehend von der Axialposition nach Figur 27 durch Einschieben der Führungshülse 2b in die Ringkappe 41 des Dosierzylinders 2a erreicht wird. In dieser weiteren Axialposition nach Fig. 30 ist eine Relativdrehung des Dosierzylinders 2a zur Führungshülse 2b um die Längsachse 7 gesperrt, die nicht rotationssymmetrisch gestalteten Drehsicherungs-Wandabschnitte 48, 49 direkt aneinander anliegen. Die Axialposition nach Fig. 30 ist axial gesichert durch eine weitere Umfangsrippe 52, die in dieser weiteren Axialposition in die Umfangsnut 51 der Ringkappe 41 eingreift.

Die Komponenten 48 bis 52 stellen gemeinsam eine Axial-/Drehsicherungseinrichtung der Dosiervorrichtung 44 dar.

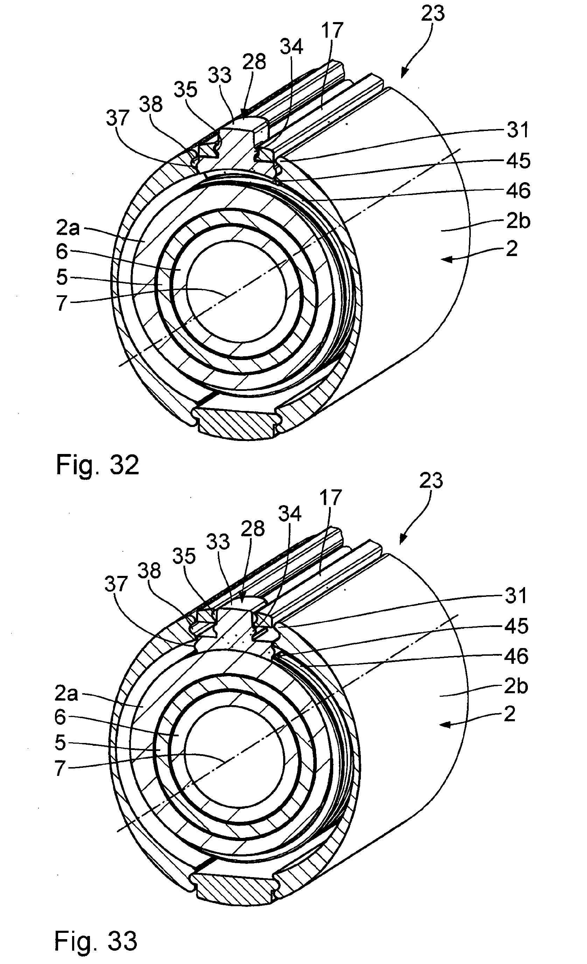

Bei der Vorgabe einer Axialposition des Anschlagkörpers 28 wird dieser bei der Dosiervorrichtung 44 zunächst vergleichbar zur Handhabung der Dosiervorrichtung 23 in eine Axialposition relativ zum Vorratsbehälter 2 grob voreingestellt und dann von der Freigabestellung, die z. B. in der Fig. 32 dargestellt ist, in die Verriegelungsstellung, die in der Fig. 33 dargestellt ist, überführt. Während dieser Vorbereitung der Axialstellung des Anschlagkörpers 28 sind der Dosierzylinder 2a und die Führungshülse 2b des Vorratsbehälters 2 in der Axialposition nach Fig. 27. Nach der groben Vorgabe der Axialposition des Anschlagkörpers 28 wird nun der Dosierzylinder 2a relativ zur Führungshülse 2b um die Längsachse 7 verdreht. Durch die Schraubwirkung der Verriegelungsstruktur 45 mit der VerriegelungsGegenstruktur 46 verlagert sich hierbei der Anschlagkörper fein in Axialrichtung relativ zur Führungshülse 2b, sodass eine Feineinstellung der Axialposition des Anschlagkörpers 28 bewirkt wird. Durch diese Gewindeverstellung kann auch eine fehlerhafte Vorgabe der Axialstellung des Anschlagkörpers 28 korrigiert werden. Nach der Einstellung der Axialposition des Anschlagkörpers 28 werden die Drehsicherungs-Wandabschnitte 48, 49 in Umfangsrichtung miteinander in Überdeckung gebracht und die Führungshülse 2b vollständig in den Dosierzylinder 2a eingeschoben. Hierbei knüpft die Umfangsrippe 50 zunächst aus der Umfangsnut 51 aus und die Führungshülse 2b wird solange in Axialrichtung in die Ringkappe 41 des Dosierzylinders 2a weiter eingeschoben, bis die Umfangsrippe 52 in die Umfangsnut 51 einrückt. Die beiden Umfangsrippen 50, 52 einerseits und die Umfangsnut 51 andererseits sind so gestaltet, dass ein Überwinden der hierdurch gebildeten Rastverbindungen in Einschubrichtung der Führungshülse 2b in die Ringkappe 41 des Dosierzylinders 2a leichter möglich ist als ein Herausziehen der Führungshülse 2a aus dem Dosierzylinder 2a. Die Rippen 50, 52 sind hierzu mit geneigtem Querschnitt ausgeführt und die Nut 51 komplementär hierzu.

Das Aufziehen des Präparats und das Verabreichen des Präparats entsprechen bei der Dosiervorrichtung 44 dem, was vorstehend zu den Dosiervorrichtungen 1 und 23 erläutert wurde.

Die Komponenten der vorstehend erläuterten Dosiervorrichtungen 1, 23 und 44 sind aus Kunststoff.

Soweit vorstehend Rastverbindungen beschrieben wurden, die durch komplementäre Rippen-/Nutgestaltungen gebildet sind, können die Rippen-/Nutgestaltungen auch jeweils vertauscht an den zugeordneten Komponenten vorgesehen sein.

Bei einer alternativen Variante ist die Dosiervorrichtung 1 als Dosierspritze mit einem als Vollkörper gestalteten Kolben 6 ausgeführt. Der Hohlraum im Inneren des Dosier-Kolbenkörpers 4 und auch die Ansaugöffnung entfallen dann. Der Dosier-Kolbenkörper 4 kann mit der äußeren Dosierhülse 5 bei dieser Variante einstückig verbunden sein. Auch die Durchgangsöffnung zwischen der Kolbenkörperöffnung 14 und der Ansaugöffnung 21, die bei den Ausführungen, die vorstehend im Zusammenhang mit den Figuren 1 bis 33 erläutert wurden, vorhanden ist, entfällt bei dieser Dosierspritzen-Variante. Die Dosierspritzen-Variante kann zur Vorgabe einer Dosierposition des Kolbens 6 im Vorratsbehälter 2 die Komponenten aufweisen, die vorstehend im Zusammenhang insbesondere mit der Rasteinrichtung 19 und der Anschlageinrichtung 27 sowie im Zusammenhang mit der Axial-/Drehsicherungseinrichtung 48 bis 52 erläutert wurden.

## Patentansprüche

1. Dosiervorrichtung (1) zur dosierten Verabreichung eines fließfähigen Präparates zusammen mit einem fließfähigen Trägermediums,
- mit einem Vorratsbehälter (2) für das Präparat, der über mindestens eine Zugangsöffnung (3) mit der Umgebung kommuniziert,
- mit einem Dosier-Kolbenkörper (4), der zumindest abschnittsweise in den Vorratsbehälter (2) abgedichtet verlagerbar ist zwischen
-- einer Ausgangsposition, in der der Dosier-Kolbenkörper (4) ganz in den Vorratsbehälter (2) eingeschoben ist und
-- mindestens einer Dosierposition, in der der Dosier-Kolbenkörper (4) bis zu einer der gewünschten Dosiermenge des Präparats entsprechenden Wegstrecke aus dem Vorratsbehälter (2) herausgeschoben ist,
- wobei der Dosier-Kolbenkörper (4) hohl ist und eine Durchgangsöffnung frei gibt zwischen
-- einer Kolbenkörperöffnung (14), die der mindestens einen Zugangsöffnung (3) zugewandt ist, und
-- einer von außen zugänglichen Ansaugöffnung (21) des Dosier-Kolbenkörpers (4),
- wobei der Dosier-Kolbenkörper (4) eine äußere Dosierhülse (5) und einen inneren, hohlen Kolbenkörper (6) aufweist, der um eine Längsachse (7) der Dosiervorrichtung (1) in der Dosierhülse (5) drehbar ist,
- wobei die Dosierhülse (5) in einem der Zugangsöffnung (3) des Vorratsbehälters (2) zugewandten Dosierhülsenboden (10) mindestens eine exzentrisch zur Längsachse (7) angeordnete Dosierhülsenöffnung (11) aufweist,
- wobei der innere Kolbenkörper (6) in einem der Zugangsöffnung (3) des Vorratsbehälters (2) zugewandten Kolbenkörperboden (12) die Kolbenkörperöffnung (14) aufweist, welche exzentrisch zur Längsachse angeordnet ist.
- wobei in einer Durchgangs-Relativ-Drehposition der Dosierhülse (5) zum inneren Kolbenköper (6) ein Durchgang zwischen dem Inneren des Vorratsbehälters (2) und dem Inneren des inneren Kolbenkörpers (6) durch die Dosierhülsenöffnung (11) und die Kolbenkörperöffnung (14) vorliegt,
- wobei in einer Verschluss-Relativ-Drehposition der Dosierhülse (5) zum inneren Kolbenkörper (6) der Durchgang zwischen dem Inneren des Vorratsbehälters (2) und dem Inneren des inneren Kolbenkörpers (6) durch die Dosierhülsenöffnung (11) und die Kolbenkörperöffnung (14) verschlossen ist.

2. Dosiervorrichtung nach Anspruch 1, **gekennzeichnet durch** eine Rasteinrichtung (19) mit mindestens einem Rasterelement (20) am Dosier-Kolbenkörper (4) und mit mindestens einem Gegen-Rasterelement am Vorratsbehälter (2) zur Vorgabe von mindestens zwei Dosier-Rasthubstellungen des Dosier-Kolbenkörpers (4) im Vorratsbehälter (2), ausgehend von der Ausgangsposition, die entsprechende Dosierposition des Dosier-Kolbenkörpers (4) vorgeben, die sich in der Wegstrecke zwischen der Ausgangsposition und der jeweiligen Dosierposition unterscheiden.

3. Dosiervorrichtung (1) nach Anspruch 2, **dadurch gekennzeichnet, dass** die Dosierhülse (5) einen inneren Dosierhülsenabschnitt (15) aufweist, der im Vorratsbehälter (2) abgedichtet geführt ist, und einen äußeren Dosierhülsenabschnitt (17), der an einer Außenwand des Vorratsbehälters (2) geführt ist, wobei die Rasteinrichtung (19) im Bereich einer Führung des äußeren Dosierhülsenabschnitts (17) am Vorratsbehälter (2) angeordnet ist.

4. Dosiervorrichtung nach einem der Ansprüche 1 bis 3, **dadurch gekennzeichnet, dass** eine Dichtung zwischen dem Dosier-Kolbenkörper (4) und dem Vorratsbehälter (2) durch eine am Dosier-Kolbenkörper (4) einstückig angeformte Dichtung (16) ausgeführt ist.

5. Dosiervorrichtung nach einem der Ansprüche 1 bis 4, **dadurch gekennzeichnet, dass** der Dosierhülsenboden (10) gegen den Kolbenkörperboden (12) über einen Dichtkörper (13) abgedichtet ist, der in der Durchgangs-Relativ-Drehposition einen Durchgang (11, 14) zwischen dem Inneren des Vorratsbehälters (2) und dem Inneren des inneren Kolbenkörpers (6) freigibt.

6. Dosiervorrichtung nach einem der Ansprüche 1 bis 5, **gekennzeichnet durch** ein Einwegeventil (13) im Durchgang (11, 14) zwischen den inneren des Vorratsbehälters (2) und dem Inneren des inneren Kolbenkörpers (6), welches einen Präparatfluss vom Vorratsbehälters (2) in den inneren Kolbenkörper (6) zulässt und einen Präparatfluss in Gegenrichtung sperrt.

7. Dosiervorrichtung nach einem der Ansprüche 5 oder 6, **dadurch gekennzeichnet, dass** der Dichtkörper (13) und/oder das Einwegeventil (13) in einer Ausnehmung (14a) im Dosierhülsenboden (10) angeordnet ist.

8. Dosiervorrichtung nach einem der Ansprüche 1 bis 7, **dadurch gekennzeichnet, dass** eine Führung einer Drehbewegung der äußeren Dosierhülse (5) zum inneren Kolbenkörper (6) durch mindestens ein teilkreisförmiges, in Umfangsrichtung um die Längsachse (7) angeordnetes Langloch (25), ausgeführt in der Dosierhülse (5) oder im Kolbenkörper (6), und einen in dem Langloch (25) laufenden Führungsstift (26), ausgebildet am Kolbenkörper (6) oder an der Dosierhülse (5), gebildet ist.

9. Dosiervorrichtung nach einem der Ansprüche 1 bis 8, **gekennzeichnet durch** eine Anschlageinrichtung (27) mit einem axial am Vorratsbehälter (2) festgelegten Anschlagkörper (28) und einem axial an der Dosierhülse (5) festgelegten Gegenanschlagkörper (29) zur Vorgabe einer Dosier-Rasthubstellung des Dosier-Kolbenkörpers (4) im Vorratsbehälter (2), die eine entsprechende Dosierstellung des Dosier-Kolbenkörpers (4), ausgehend von einer eingeschobenen Ausgangsstellung des Dosier-Kolbenkörpers (4) im Vorratsbehälter (2), darstellt.

10. Dosiervorrichtung nach Anspruch 9, **dadurch gekennzeichnet, dass** die Dosierhülse (5) einen inneren Dosierhülsenabschnitt (15) aufweist, der im Vorratsbehälter (2) abgedichtet geführt ist, und einen äußeren Dosierhülsenabschnitt (17), der an einer Außenwand (31) des Vorratsbehälters (2) geführt ist, wobei der Anschlagkörper (28) im Bereich einer Führung des äußeren Dosierhülsenabschnitts (17) am Vorratsbehälter (2) angeordnet ist.

11. Dosiervorrichtung nach Anspruch 9 oder 10, **dadurch gekennzeichnet, dass** der Anschlagkörper (28) in einem Langloch (30) der Dosierhülse (5) zur Vorgabe der Dosierstellung verschiebbar ist.

12. Dosiervorrichtung nach einem der Ansprüche 9 bis 11, **dadurch gekennzeichnet, dass** der Anschlagkörper (28) eine Verriegelungsstruktur (32) aufweist und verlagerbar ist zwischen einer Freigabestellung, in der die Verriegelungsstruktur (32) wirkungslos ist, und einer Verriegelungsstellung, in der die Verriegelungsstruktur (32) mit einer komplementären Verriegelungs-Gegenstruktur (36) zusammenwirkt, die am Vorratsbehälter (2) ausgeführt ist, sodass in der Verriegelungsstellung der Anschlagkörper (28) axial am Vorratsbehälter (2) festgelegt ist.

13. Dosiervorrichtung nach Anspruch 12, **gekennzeichnet durch** eine erste Rasteinrichtung (34, 35), die den Anschlagkörper (28) überwindbar in der Freigabestellung sichert, und eine zweite Rasteinrichtung (37, 38), die den Anschlagkörper (28) nach Überwindung der ersten Rasteinrichtung (34, 35) in der Verriegelungsstellung sichert.

14. Dosiervorrichtung nach einem der Ansprüche 1 bis 13, **dadurch gekennzeichnet, dass** der Vorratsbehälter (2) zweiteilig ist und einen inneren Dosierzylinder (2a) mit einem Vorratsvolumen und eine äußere Führungshülse (2b) aufweist, wobei der Dosierzylinder (2a) und die Führungshülse (2b) axial und, in einer axialen Relativ-Endstellung, gegen eine Relativdrehung zueinander um die Längsachse (7) aneinander gesichert sind.

15. Dosiervorrichtung nach Anspruch 14, **gekennzeichnet durch** eine Axial/Drehsicherungseinrichtung (48 bis 52), die in einer ersten Axialposition des Dosierzylinders (2a) zur Führungshülse (2b) eine Relativdrehung des Dosierzylinders (2a) zur Führungshülse (2b) um die Längsachse (7) zulässt und in einer zweiten Axialposition des Dosierzylinders (2a) zur Führungshülse (2b) eine Relativdrehung des Dosierzylinders (2a) zur Führungshülse (2b) um die Längsachse (7) sperrt, wobei über eine Relativdrehung des Dosierzylinders (2a) zur Führungshülse (2b) in der ersten Axialposition eine Feinvorgabe einer Axialposition des Anschlagkörpers (28) zum Dosierzylinder (2a) und damit der Dosierposition des Dosier-Kolbenkörper (4) im Vorratsbehälter (2), vorgebbar ist.

## Claims

1. Metering device (1) for the metered administration of a flowable preparation together with a flowable carrier medium,
- with a storage container (2) for the preparation, which communicates with the environment via at least one access opening (3),
- with a metering plunger body (4), which is displaceable in a sealed manner at least in sections in the storage container (2) between
-- a starting position, in which the metering plunger body (4) is pushed fully into the storage container (2) and
-- at least one metering position, in which the metering plunger body (4) is pushed out of the storage container (2) by a distance corresponding to the desired metering amount,
- wherein the metering plunger body (4) is hollow and releases a through opening between
-- a plunger body opening (14), which faces the at least one access opening (3), and
-- a suction opening (21) of the metering plunger body (4) accessible from the outside,
- wherein the metering plunger body (4) has an outer metering sleeve (5) and an inner, hollow plunger body (6), which is rotatable about a longitudinal axis (7) of the metering device (1) in the metering sleeve (5),
- wherein the metering sleeve (5) in a metering sleeve bottom (10) facing the access opening (3) of the storage container (2) has at least one metering sleeve opening (11) arranged eccentrically to the longitudinal axis (7),
- wherein the inner plunger body (6) in a plunger body bottom (12) facing the access opening (3) of the storage container (2) comprises the plunger body opening (14), which is arranged eccentrically to the longitudinal axis,
- wherein in a passage relative rotational position of the metering sleeve (5) relative to the inner plunger body (6) there is a passage between the inside of the storage container (2) and the inside of the inner plunger body (6) through the metering sleeve opening (11) and the plunger body opening (14),
- wherein in a closed relative rotational position of the metering sleeve (5) relative to the inner plunger body (6) the passage between the inside of the storage container (2) and the inside of the inner plunger body (6) through the metering sleeve opening (11) and the plunger body opening (14) is closed.

2. Metering device according to claim 1, **characterized by** a locking device (19) with at least one locking element (20) on the metering plunger body (4) and with at least one counter locking element on the storage container (2) for defining at least two metering locking lift positions of the metering plunger body (4) in the storage container (2), from the starting position, which positions define the corresponding metering position of the metering plunger body (4) and differ in the distance between the starting position and the respective metering position.

3. Metering device (1) according to claim 2, **characterized in that** the metering sleeve (5) comprises an inner metering sleeve section (15), which is guided in a sealed manner in the storage container (2), and an outer metering sleeve section (17), which is guided on an outer wall of the storage container (2), wherein the locking device (19) is arranged in the area of a guide of the outer metering sleeve section (17) on the storage container (2).

4. Metering device according to one of claims 1 to 3, **characterized in that** a seal is formed between the metering plunger body (4) and the storage container (2) by a seal (16) formed in one piece on the metering plunger body (4).

5. Metering device according to one of claims 1 to 4, **characterized in that** the metering sleeve bottom (10) is sealed against the plunger body base (12) by a sealing body (13), which in the through relative rotational position provides access (11, 14) between the inside of the storage container (2) and the inside of the inner plunger body (6).

6. Metering device according to one of claims 1 to 5, **characterized by** a one-way valve (13) in the passage (11, 14) between the inside of the storage container (2) and the inside of the inner plunger body (6), which allows the flow of preparation from the storage container (2) into the inner plunger body (6) and blocks the flow of preparation in opposite direction.

7. Metering device according to one of claims 5 or 6, **characterized in that** the sealing body (13) and/or one-way valve (13) is arranged in a recess (14a) in the metering sleeve bottom (10).

8. Metering device according to one of claims 1 to 7, **characterized in that** a guide of a rotational movement of the outer metering sleeve (5) to the inner plunger body (6) is formed by at least one partly circular elongated hole (25) arranged in circumferential direction about the longitudinal axis (7), formed in the metering sleeve (5) or in the plunger body (6) and a guiding pin (26) running in the elongated hole (25) formed on the plunger body (6) or on the metering sleeve (5).

9. Metering device according to one of claims 1 to 8, **characterized by** a stop device (27) with a stop body (28) secured axially on the storage container (2) and a counter stop body (29) secured axially on the metering sleeve (5) for determining a metering lock lift position of the metering plunger body (4) in the storage container (2), which represents a corresponding metering position of the metering plunger body (4), starting from an inserted starting position of the metering plunger body (4) in the storage container (2).

10. Metering device according to claim 9, **characterized in that** the metering sleeve (5) has an inner metering sleeve section (15), which is guided in a sealed manner in the storage container (2), and an outer metering sleeve section (17), which is guided on an outer wall (31) of the storage container (2), wherein the stop body (28) is arranged in the area of a guide of the outer metering sleeve section (17) on the storage container (2).

11. Metering device according to claim 9 or 10, **characterized in that** the stop body (28) can be displaced in an elongated hole (30) of the metering sleeve (5) for determining the metering position.

12. Metering device according to one of claims 9 to 11, **characterized in that** the stop body (28) has a locking structure (32) and is displaceable between a release position, in which the locking structure (32) is ineffective, and a locking position, in which the locking structure (32) cooperates with a complementary locking counter structure (36), which is formed on the storage container (2), so that in the locking position the stop body (28) is secured axially on the storage container (2).

13. Metering device according to claim 12, **characterized by** a first locking device (34, 35), which secures the stop body (28) surmountably in the release position, and a second locking device (37, 38), which secures the stop body (28) after surmounting the first locking device (34, 35) in the locking position.

14. Metering device according to one of claims 1 to 13, **characterized in that** the storage container (2) is in two parts and comprises an inner metering cylinder (2a) with a supply volume and an outer guiding sleeve (2b), wherein the metering cylinder (2a) and the guiding sleeve (2b) are secured axially to one another and, in an axial relative end position, against relative rotation to one another about the longitudinal axis (7).

15. Metering device according to claim 14, **characterized by** an axial/rotation protection device (48 to 52), which in a first axial position of the metering cylinder (2a) to the guiding sleeve (2b) allows a relative rotation of the metering cylinder (2a) to the guiding sleeve (2b) about the longitudinal axis (7) and in a second axial position of the metering cylinder (2a) to the guiding sleeve (2b) blocks a relative rotation of the metering cylinder (2a) to the guiding sleeve (2b) about the longitudinal axis (7), wherein by means of a relative rotation of the metering cylinder (2a) to the guiding sleeve (2b) in the first axial position a fine definition can be made of an axial position of stop body (28) to the metering cylinder (2a) and thereby the metering position of the metering plunger body (4) in the storage container (2).

## Revendications

1. Dispositif de dosage (1), pour l'administration dosée d'une préparation fluide, conjointement avec un véhicule fluide,
- comprenant un réservoir (2), destiné à la préparation, qui communique avec l'environnement par au moins une ouverture d'accès (3),
- comprenant un corps de piston doseur (4), qui peut être déplacé de manière étanche au moins partiellement dans le réservoir (2) entre
-- une position de départ, dans laquelle le corps de piston doseur (4) est totalement enfoncé dans le réservoir (2), et
-- au moins une position de dosage, dans laquelle le corps de piston doseur (4) est sorti du réservoir (2) d'une course correspondant à la quantité dosée souhaitée de la préparation,
- le corps de piston doseur (4) étant creux et offrant une ouverture pour le passage entre
-- une ouverture du corps de piston (14), qui est orientée vers au moins une ouverture d'accès (3), et
-- une ouverture d'aspiration (21) du corps de piston doseur (4), accessible de l'extérieur,
- le corps de piston doseur (4) présentant une enveloppe de dosage (5) externe et un corps de piston (6) interne, creux, qui peut être mis en rotation dans l'enveloppe de dosage (5) autour d'un axe longitudinal (7) du dispositif de dosage (1),
- l'enveloppe de dosage (5) présentant au moins une ouverture d'enveloppe de dosage (11), disposée de manière excentrique par rapport à l'axe longitudinal (7) dans un fond (10) de l'enveloppe de dosage orienté vers l'ouverture d'accès (3) du réservoir (2),
- le corps de piston (6) interne présentant une ouverture de corps de piston (14), laquelle est disposée de manière excentrique par rapport à l'axe longitudinal, dans le fond (12) du corps de piston orienté vers l'une des ouvertures d'accès (3) du réservoir (2),
- un passage, vers le corps de piston (6) interne, entre l'intérieur du réservoir (2) et l'intérieur du corps de piston intérieur (6), traversant l'ouverture d'enveloppe de dosage (11) et l'ouverture du corps de piston (14), étant présent dans une position de rotation relative de passage de l'enveloppe de dosage (15),
- le passage entre l'intérieur du réservoir (2) et l'intérieur du corps de piston (6) interne, traversant l'ouverture de l'enveloppe de dosage (11) et l'ouverture du corps de piston (14), étant fermé dans une position de rotation relative de fermeture de l'enveloppe de dosage (5) par rapport au corps de piston (6) interne.

2. Dispositif de dosage selon la revendication 1, **caractérisé par** un système d'encliquetage (19) comprenant au moins un élément d'encliquetage (20) sur le corps du piston doseur (4) et comprenant au moins un élément d'encliquetage complémentaire sur le réservoir (2), pour la prédéfinition d'au moins deux positions de course de l'encliquetage de dosage du corps de piston doseur (4) dans le réservoir (2) en partant de la position de départ, qui prédéterminent la position de dosage du corps de piston doseur (4) correspondante, qui se différentient dans la course entre la position de départ et la position de dosage correspondante.

3. Dispositif de dosage (1) selon la revendication 2, **caractérisé en ce que** l'enveloppe de dosage (5) présente une portion interne (15) d'enveloppe de dosage, qui est menée de manière étanche dans le réservoir (2), et une portion externe (17) d'enveloppe de dosage, qui est menée sur une face externe du réservoir (2), le système d'encliquetage (19) étant disposé sur le réservoir (2) au niveau d'un guide de la portion externe (17) de l'enveloppe de dosage.

4. Dispositif de dosage selon l'une des revendications 1 à 3, **caractérisé en ce qu'**un joint entre le corps de piston doseur (4) et le réservoir (2) est conçu sous la forme d'un joint (16) surmoulé sur le corps du piston doseur (4), formant une pièce unique.

5. Dispositif de dosage selon l'une des revendications 1 à 4, **caractérisé en ce que** le fond (10) de l'enveloppe de dosage est rendu étanche vis-à-vis du fond (12) du corps de piston par l'intermédiaire d'un organe d'étanchéité (13) qui, dans la position de rotation relative au passage, libère un passage (11, 14) entre l'intérieur du réservoir (2) et l'intérieur du corps de piston (6) interne.

6. Dispositif de dosage selon l'une des revendications 1 à 5, **caractérisé par** une valve anti-reflux (13) dans le passage (11, 14) entre l'intérieur du réservoir (2) et l' intérieur du corps de piston (6) interne, laquelle permet un flux de préparation du réservoir (2) dans le corps de piston (6) interne et empêche un flux de préparation dans le sens opposé.

7. Dispositif de dosage selon l'une des revendications 5 ou 6, **caractérisé en ce que** l'organe d'étanchéité (13) et/ou la valve anti-reflux (13) sont disposés dans un évidement (14a) dans le fond (10) de l'enveloppe de dosage.

8. Dispositif de dosage selon l'une des revendications 1 à 7, **caractérisé en ce qu**'un guide d'un mouvement de rotation de l'enveloppe de dosage (5) externe vers le corps de piston (6) interne est formé sur le corps de piston (6) ou sur l'enveloppe de dosage (5) par au moins un orifice oblong (25), en forme d'arc de cercle, disposé dans la direction circonférentielle autour de l'axe longitudinal (7), conçu dans l'enveloppe de dosage (5) ou dans le corps de piston (6), et d'une tige de guidage (26) parcourant l'orifice oblong (25) formé sur le corps de piston (6) ou sur l'enveloppe de dosage (5).

9. Dispositif de dosage selon l'une des revendications 1 à 8, **caractérisé par** un système de butée (27), comprenant un organe de butée (28) fixé axialement sur le réservoir (2) et un organe de butée complémentaire (29) fixé sur l'enveloppe de dosage (5), pour le préréglage d'une position de course d'encliquetage de dosage du corps de piston doseur (4) dans le réservoir (2), qui représente une position de dosage du corps de piston doseur (4) correspondante, en partant d'une position de départ du corps de piston doseur (4) rentrée dans le réservoir (2).

10. Dispositif de dosage selon la revendication 9, **caractérisé en ce que** l'enveloppe de dosage (5) présente une portion interne (15) d'enveloppe de dosage, qui est menée dans le réservoir (2) de manière étanche, et une portion externe (17) d'enveloppe de dosage, qui est menée sur la face extérieure (31) du réservoir (2), l'organe de butée (28) étant disposé dans la zone d'un guide de la portion externe (17) d'enveloppe de dosage sur le réservoir (2).

11. Dispositif de dosage selon les revendications 9 ou 10, **caractérisé en ce que** le corps de butée (28) peut être déplacé dans un orifice oblong (30) de l'enveloppe de dosage (5) pour le préréglage de la position de dosage.

12. Dispositif de dosage selon l'une des revendications 9 à 11, **caractérisé en ce que** l'organe de butée (28) présente une structure de verrouillage (32) et peut être déplacé entre une position de libération, dans laquelle la structure de verrouillage (32) est sans effet, et une position de verrouillage, dans laquelle la structure de verrouillage (32) agit conjointement avec une structure complémentaire de verrouillage (36), qui est implantée sur le réservoir (2), de sorte que l'organe de butée (28) est fixé axialement sur le réservoir (2) dans la position de verrouillage.

13. Dispositif de dosage selon la revendication 12, **caractérisé par** un premier système d'encliquetage (34, 35), qui sécurise l'organe de butée (28) de manière insurmontable dans la position de libération, et un deuxième système d'encliquetage (37, 38) qui sécurise l'organe de butée (28) dans la position de verrouillage après avoir surmonté le premier système d'encliquetage (34, 35).

14. Dispositif de dosage selon l'une des revendications 1 à 13, **caractérisé en ce que** le réservoir (2) est en deux parties et présente un cylindre de dosage interne (2a), comprenant un volume de réserve, et une enveloppe de guidage externe (2b), le cylindre de dosage (2a) et l'enveloppe de guidage (2b) étant sécurisés axialement et l'un par rapport à l'autre, dans une position finale relative axiale, contre une rotation relative de l'un par rapport à l'autre autour de l'axe longitudinal (7).

15. Dispositif de dosage selon la revendication 14, **caractérisée par** un système de protection contre la rotation (48 à 52) axial qui permet une rotation relative du cylindre de dosage (2a) par rapport à l'enveloppe de guidage (2b) autour de l'axe longitudinal (7) dans une première position axiale du cylindre de dosage (2a) par rapport à l'enveloppe de guidage (2b), et empêche une rotation relative du cylindre de dosage (2a) par rapport à l'enveloppe de guidage (2b) autour de l'axe longitudinal (7) dans une deuxième position axiale du cylindre de dosage (2a) par rapport à l'enveloppe de guidage (2b), où, par l'intermédiaire d'une rotation relative du cylindre de dosage (2a) par rapport à l'enveloppe de guidage (2a) dans la première position axiale, un préréglage précis d'une position axiale de l'organe de butée (28) par rapport au cylindre de dosage (2a), et ainsi, d'une position de dosage du corps de piston doseur (4) dans le réservoir (2), peut être préréglé.
